# EUROPEAN PATENT SPECIFICATION

(11) **EP 4 194 552 B1**
(45) Date of publication and mention of the grant of the patent: **03.06.2026**
(21) Application number: 21854391.6
(22) Date of filing: 30.07.2021
(51) Int. Cl.: C12N 15/11, C12N 15/115, C12Q 1/6804, G01N 33/53

(54) **NUCLEIC ACID STRUCTURE**
NUKLEINSÄURESTRUKTUR
STRUCTURE D'ACIDE NUCLÉIQUE

(30) Priority: 05.08.2020 JP 2020132829
(43) Date of publication of application: 14.06.2023
(73) Proprietor: Cranebio Co., Ltd., Tokyo, 101-0021 (JP); The School Corporation Kansai University, Suita-shi, Osaka 564-8680 (JP)
(72) Inventor: KUZUYA, Akinori, Suita-shi, Osaka 565-0841 (JP); TAKAHASHI, Nozomu, Suita-shi, Osaka 564-0061 (JP); OKAMOTO, Yuta, Suita-shi, Osaka 565-0842 (JP); KONO, Sachiko, Ibaraki-shi, Osaka 567-0031 (JP); KATO, Chihiro, Osaka-shi, Osaka 533-0004 (JP); SAITO, Keita, Tokyo 113-0021 (JP)
(74) Representative: Müller-Boré & Partner Patentanwälte PartG mbB
(86) International application number: PCT/JP2021/028281
(87) International publication number: WO 2022/030378

(56) References cited:
- HEIDI-KRISTIN WALTER ET AL: ""DNA Origami Traffic Lights" with a Split Aptamer Sensor for a Bicolor Fluorescence Readout", NANO LETTERS,20200311AMERICAN CHEMICAL SOCIETY, US, vol. 17, no. 4, 8 March 2017 (2017-03-08), pages 2467 - 2472, XP055730340, ISSN: 1530-6984, DOI: 10.1021/acs.nanolett.7b00159
- HOU TING ET AL: "Label-free colorimetric detection of coralyne utilizing peroxidase-like split G-quadruplex DNAzyme", vol. 5, no. 18, 1 January 2013 (2013-01-01), GB, pages 4671, XP055905447, ISSN: 1759-9660, Retrieved from the Internet <URL:https://pubs.rsc.org/en/content/articlepdf/2013/ay/c3ay40796a> DOI: 10.1039/c3ay40796a
- AKINORI KUZUYA ET AL: "Nanomechanical DNA origami 'single-molecule beacons' directly imaged by atomic force microscopy", NATURE COMMUNICATIONS, vol. 2, 23 August 2011 (2011-08-23), pages 449, XP055133811, DOI: 10.1038/ncomms1452
- TELLER C ET AL: "Organizing protein-DNA hybrids as nanostructures with programmed functionalities", TRENDS IN BIOTECHNOLOGY, ELSEVIER PUBLICATIONS, CAMBRIDGE, GB, vol. 28, no. 12, 1 December 2010 (2010-12-01), pages 619 - 628, XP027483990, ISSN: 0167-7799, [retrieved on 20101028], DOI: 10.1016/J.TIBTECH.2010.09.005
- WALTER HK ET AL.: "DNA Origami Traffic Lights", SPLIT APTAMER SENSOR FOR A BICOLOR FLUORESCENCE READOUT, NANO LETT., vol. 17, no. 4, 2017, pages 2467 - 2472, XP055730340, DOI: 10.1021/acs.nanolett.7b00159
- HOU TING, LI CHUNXIANG, WANG XIUZHONG, ZHAO CAN, LI FENG: "Label-free colorimetric detection of coralyne utilizing peroxidase-like split G-quadruplex DNAzyme", ANALYTICAL METHODS, ROYAL SOCIETY OF CHEMISTRY, GB, vol. 5, no. 18, 1 January 2013 (2013-01-01), GB , pages 4671, XP055905447, ISSN: 1759-9660, DOI: 10.1039/c3ay40796a
- AKINORI KUZUYA, YUSUKE SAKAI, TAKAHIRO YAMAZAKI, YAN XU, MAKOTO KOMIYAMA: "Nanomechanical DNA origami 'single-molecule beacons' directly imaged by atomic force microscopy", NATURE COMMUNICATIONS, vol. 2, pages 449, XP055133811, DOI: 10.1038/ncomms1452

## Description

### Technical Field

The present invention relates to a nucleic acid structure and the like.

### Background Art

Techniques for detecting nucleic acids, proteins, peptides, low-molecular-weight compounds, metal ions, and the like are used in various fields, such as medical and environmental fields. For example, disease tests may be performed using the presence and amount of specific nucleic acids and other biomolecules as indicators.

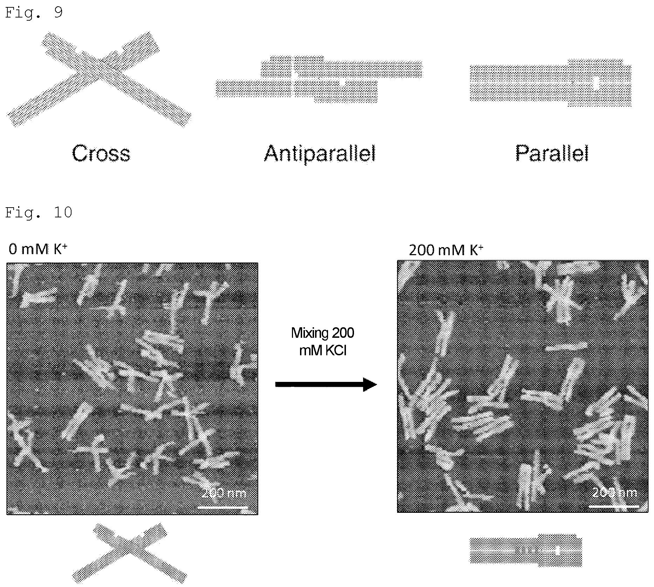

On the other hand, DNA origami is a nucleic acid structure formed by folding single-stranded circular DNA into many short DNA strands (staple DNAs) that can complementarily bind to the DNA. Various structures can be created at will, depending on the design of the sequence of staple DNA. NPL 1 has reported DNA origami (DNA origami pliers (or DNA pliers)) having a structure in which two levers are joined at a fulcrum (Holliday junction). DNA origami pliers include three possible structures (Fig. 9: antiparallel, cross, and parallel types) based on the conformation of the Holliday junction. So far, there have been reports on techniques for detecting Na+, Ag+, streptavidin, IgG, ATP, and miRNA using structural changes of DNA origami pliers. However, it is necessary for these techniques to fluorescently detect small fluorescence intensity changes based on structural changes, or to detect structural changes by an atomic force microscope.

### Citation List

### Non-patent Literature

NPL 1: Kuzuya, A., (2011) Nat. Commun. doi: 10.1038/ncomms1452

### Summary of Invention

### Technical Problem

An object of the present invention is to provide a technique for detecting a test substance more easily using a nucleic acid structure.

### Solution to Problem

As a result of intensive research in view of the above object, the present inventors found that the object can be achieved by a nucleic acid structure comprising a partial nucleic acid structure 1 and a partial nucleic acid structure 2,
the partial nucleic acid structure 1 and the partial nucleic acid structure 2 being linked by a Holliday junction,
the partial nucleic acid structure 1 comprising a protruding structure 1a containing a nucleic acid aptamer sequence, and a protruding structure 1b containing a split domain 1 of an enzyme, and
the partial nucleic acid structure 2 comprising a protruding structure 2a containing a sequence complementary to part or all of the nucleic acid aptamer sequence, and a protruding structure 2b containing a split domain 2 paired with the split domain 1.
Upon further research based on this finding, the present inventors have completed the present invention. More specifically, the present invention includes the following embodiments.

Item 1. A nucleic acid structure comprising a partial nucleic acid structure 1 and a partial nucleic acid structure 2,
the partial nucleic acid structure 1 and the partial nucleic acid structure 2 being linked by a Holliday junction,
the partial nucleic acid structure 1 comprising a protruding structure 1a containing a nucleic acid aptamer sequence, and a protruding structure 1b containing a split domain 1 of an enzyme, and
the partial nucleic acid structure 2 comprising a protruding structure 2a containing a sequence complementary to part or all of the nucleic acid aptamer sequence, and a protruding structure 2b containing a split domain 2 paired with the split domain 1.

Item 2. The nucleic acid structure according to Item 1, wherein the partial structure 1 and the partial structure 2 each have an elongated shape.

Item 3. The nucleic acid structure according to Item 1 or 2, which is made of a material containing a single-stranded circular nucleic acid and a staple nucleic acid containing a sequence complementary to the single-stranded circular nucleic acid.

Item 4. The nucleic acid structure according to any one of Items 1 to 3, wherein the protruding structures are arranged so that, among three structures (structural type 1, structural type 2, and structural type 3: provided that the structural type 2 is an intermediate structure that mediates structural changes between the structural type 1 and the structural type 3) based on the conformation of the Holliday junction, when taking the structural type 1, the protruding structure 1a and the protruding structure 2a face each other, and when taking the structural type 3, the protruding structure 1b and the protruding structure 2b face each other.

Item 5. The nucleic acid structure according to Item 4, wherein the protruding structures are arranged so that the protruding structure 1a and the protruding structure 2a face each other and bind together based on complementary base pairing in the absence of a recognition substance for the nucleic acid aptamer sequence.

Item 6. The nucleic acid structure according to Item 4 or 5, wherein the protruding structures are arranged so that the protruding structure 1b and the protruding structure 2b face each other to form the enzyme in the presence of a recognition substance for the nucleic acid aptamer sequence and in the presence of a monovalent cation.

Item 7. The nucleic acid structure according to any one of Items 1 to 6, wherein the enzyme is a nucleic acid enzyme containing two or more G-quartets.

Item 8. The nucleic acid structure according to any one of Items 1 to 7, wherein the recognition substance for the nucleic acid aptamer sequence is a biological material.

Item 9. The nucleic acid structure according to any one of Items 1 to 8, wherein the recognition substance for the nucleic acid aptamer sequence is at least one member selected from the group consisting of nucleic acids, proteins, peptides, low-molecular-weight compounds, physiologically active substances, and metal ions.

Item 10. The nucleic acid structure according to any one of Items 1 to 9, which is a nano-structure.

Item 11. A test substance detection composition comprising the nucleic acid structure according to any one of Items 1 to 10.

Item 12. The test substance detection composition according to Item 11, which is a reagent or a test agent.

Item 13. A method for detecting a test substance, comprising bringing the nucleic acid structure according to any one of Items 1 to 10 into contact with a sample that may contain the test substance.

### Advantageous Effects of Invention

According to the present invention, a technique for detecting a test substance more easily using a nucleic acid structure can be provided.

### Brief Description of Drawings

Fig. 1 shows the overall view of the nucleic acid structure of Reference Example 1.
Fig. 2 shows the leftmost diagram among the six diagrams obtained by dividing Fig. 1 into six parts. The sequences of single-stranded circular DNA and staple DNAs, and staple numbers are shown. The marker parts indicate staple DNAs. The black bar indicates the boundary with the adjacent divided diagram.
Fig. 3 shows the second diagram from the left among the six diagrams obtained by dividing Fig. 1 into six parts. The sequences of single-stranded circular DNA and staple DNAs, and staple numbers are shown. The marker parts indicate staple DNAs. The black bar indicates the boundary with the adjacent divided diagram.
Fig. 4 shows the third diagram from the left among the six diagrams obtained by dividing Fig. 1 into six parts. The sequences of single-stranded circular DNA and staple DNAs, and staple numbers are shown. The marker parts indicate staple DNAs. The black bar indicates the boundary with the adjacent divided diagram.
Fig. 5 shows the fourth diagram from the left among the six diagrams obtained by dividing Fig. 1 into six parts. The sequences of single-stranded circular DNA and staple DNAs, and staple numbers are shown. The marker parts indicate staple DNAs. The black bar indicates the boundary with the adjacent divided diagram.
Fig. 6 shows the fifth diagram from the left among the six diagrams obtained by dividing Fig. 1 into six parts. The sequences of single-stranded circular DNA and staple DNAs, and staple numbers are shown. The marker parts indicate staple DNAs. The black bar indicates the boundary with the adjacent divided diagram.
Fig. 7 shows the sixth (rightmost) diagram from the left among the six diagrams obtained by dividing Fig. 1 into six parts. The sequences of single-stranded circular DNA and staple DNAs, and staple numbers are shown. The marker parts indicate staple DNAs. The black bar indicates the boundary with the adjacent divided diagram.
Fig. 8 shows a schematic diagram showing only single-stranded circular DNA in a linear form in the nucleic acid structure of Reference Example 1.
Fig. 9 shows three structures based on the conformation of the Holliday junction in the nucleic acid structure of Reference Example 2.
Fig. 10 shows AFM observation images showing structural changes due to the addition of K⁺ in the nucleic acid structure of Reference Example 2.
Fig. 11 shows changes in the proportion of each structure due to the addition of K⁺ in the nucleic acid structure of Reference Example 2.
Fig. 12 shows a photographic image of the detection results in Reference Example 3.
Fig. 13 shows a schematic diagram of structural changes in the nucleic acid structure of Example 1.
Fig. 14 shows AFM observation images showing structural changes due to the addition of miR-20 in the nucleic acid structure of Example 1.
Fig. 15 shows changes in the proportion of each structure due to the addition of miR-20 in the nucleic acid structure of Example 1.
Fig. 16 shows a photographic image of the detection results in Example 2.
Fig. 17 shows a schematic diagram of structural changes in the nucleic acid structure of Example 3.
Fig. 18 shows AFM observation images showing structural changes due to the addition of R509 in the nucleic acid structure of Example 3.
Fig. 19 shows changes in the proportion of each structure due to the addition of R509 in the nucleic acid structure of Example 3.
Fig. 20 shows a photographic image of the detection results in Example 4.
Fig. 21 shows a schematic diagram of structural changes in the nucleic acid structure of Example 5.
Fig. 22 shows AFM observation images showing structural changes due to the addition of N1 in the nucleic acid structure of Example 5.
Fig. 23 shows changes in the proportion of each structure due to the addition of N1 in the nucleic acid structure of Example 5.
Fig. 24 shows a photographic image of the detection results in Example 6.
Fig. 25 shows a schematic diagram of structural changes in the nucleic acid structure of Example 7.
Fig. 26 shows AFM observation images showing structural changes due to the addition of thrombin in the nucleic acid structure of Example 7.
Fig. 27 shows changes in the proportion of each structure due to the addition of thrombin in the nucleic acid structure of Example 7.
Fig. 28 shows a photographic image of the detection results in Example 8.
Fig. 29 shows an electrophoretic image of LgBiT and LgBiT-138w of Example 9.
Fig. 30 shows a schematic diagram of structural changes in the nucleic acid structure of Example 9.
Fig. 31 shows AFM observation images showing structural changes due to the addition of miR-20 in the nucleic acid structure of Example 9.
Fig. 32 shows changes in the proportion of each structure due to the addition of miR-20 in the nucleic acid structure of Example 9.
Fig. 33 shows the detection results in Example 10.

### Description of Embodiments

### 1. Definition

In the present specification, the terms "comprise" and "contain" include the concepts of "comprising," "containing," "consisting essentially of," and "consisting of."

In the present specification, the "identity" of base sequences refers to the degree of matching between two or more comparable base sequences. Therefore, the higher the match between two base sequences, the higher the identity or similarity of those sequences. The level of identity between base sequences is determined, for example, using a sequence analysis tool, FASTA, with default parameters. Alternatively, the identity level can be determined using the algorithm BLAST by Karlin and Altschul (Karlin S, Altschul SF. "Methods for assessing the statistical significance of molecular sequence features by using general scoring schemes" Proc Natl Acad Sci USA. 87: 2264-2268 (1990), Karlin S, Altschul SF. "Applications and statistics for multiple high-scoring segments in molecular sequences." Proc Natl Acad Sci USA. 90: 5873-7 (1993)). A program called BLASTX has been developed based on the BLAST algorithm. Specific methods of these analysis methods are known, and reference may be made to the website of the National Center of Biotechnology Information (NCBI) (http://www.ncbi.nlm.nih.gov/).

In the present specification, nucleic acids, such as DNA and RNA, may be subjected to known chemical modification, as shown below. In order to prevent degradation by hydrolases, such as nucleases, the phosphate residue (phosphate) of each nucleotide can be replaced by a chemically modified phosphate residue, such as phosphorothioate (PS), methylphosphonate, or phosphorodithionate. The hydroxyl group at position 2 of the sugar (ribose) of each ribonucleotide may be replaced by -OR (R is, for example, CH3(2'-O-Me), CH2CH2OCH3(2'-O-MOE), CH2CH2NHC(NH)NH2, CH2CONHCH3, or CH2CH2CN). Further, a base moiety (pyrimidine, purine) may be chemically modified, and examples include introduction of a methyl group or a cationic functional group into position 5 of the pyrimidine base, substitution of the carbonyl group at position 2 with thiocarbonyl, and the like. Other examples include, but are not limited to, those in which the phosphate moiety or hydroxyl moiety is modified with biotin, an amino group, a lower alkylamine group, an acetyl group, or the like. In addition, BNA (LNA), in which the conformation of the sugar moiety is fixed at the N-type by bridging the 2'oxygen and 4'carbon of the sugar moiety of the nucleotide, can also be used.

### 2. Nucleic Acid Structure

The present invention relates to a nucleic acid structure comprising a partial nucleic acid structure 1 and a partial nucleic acid structure 2,
the partial nucleic acid structure 1 and the partial nucleic acid structure 2 being linked by a Holliday junction,
the partial nucleic acid structure 1 comprising a protruding structure 1a containing a nucleic acid aptamer sequence, and a protruding structure 1b containing a split domain 1 of an enzyme, and
the partial nucleic acid structure 2 comprising a protruding structure 2a containing a sequence complementary to part or all of the nucleic acid aptamer sequence, and a protruding structure 2b containing a split domain 2 paired with the split domain 1 (also referred to as "the nucleic acid structure of the present invention" in the present specification). This is described below.

The nucleic acid structure is not particularly limited as long as it mainly contains a nucleic acid. The content of the nucleic acid in the nucleic acid structure is, for example, 70 mass% or more, preferably 80 mass% or more, more preferably 90 mass% or more, even more preferably 95 mass% or more, still more preferably 98 mass% or more, still even more preferably 99 mass% or more, and particularly preferably 100 mass%. Substances other than the nucleic acid in the nucleic acid structure are not particularly limited, and examples include proteins, peptides, sugars, labeling substances, metal ions, and the like.

The nucleic acid structure may be a three-dimensional structure, a two-dimensional structure (planer structure), or a combination thereof.

The nucleic acid structure is preferably formed by folding a nucleic acid. In terms of being suitable for the formation of the nucleic acid structure of the present invention, the nucleic acid structure is preferably a structure (DNA origami) made of a material containing a single-stranded circular nucleic acid and a staple nucleic acid containing a sequence complementary to the single-stranded circular nucleic acid.

The single-stranded circular nucleic acid that can form the nucleic acid structure is not particularly limited as long as it can be folded to form DNA origami. A single-stranded circular nucleic acid that may be composed of any base sequence can form DNA origami by designing the sequence of staple DNA according thereto. The number of bases in the single-stranded circular nucleic acid is not particularly limited, and is, for example, 500 to 50000, preferably 1000 to 25000, more preferably 2000 to 15000, even more preferably 3000 to 12000, and still more preferably 4000 to 10000. The base sequence of the single-stranded circular nucleic acid is not particularly limited, and is, for example, a base sequence containing a M13 bacteriophage DNA sequence or a sequence derived from the DNA sequence. Specific examples of the base sequence include a base sequence containing a base sequence represented by SEQ ID NO: 273, and a base sequence containing a base sequence having 70% or more (preferably 80% or more, more preferably 90% or more, even more preferably 95% or more, still more preferably 98% or more, and still even more preferably 99% or more) identity to the base sequence represented by SEQ ID NO: 273.

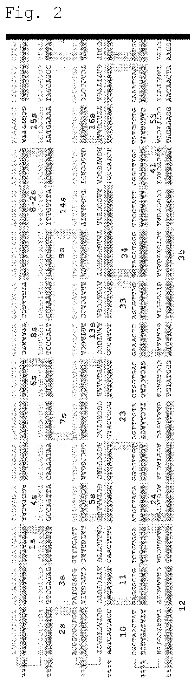

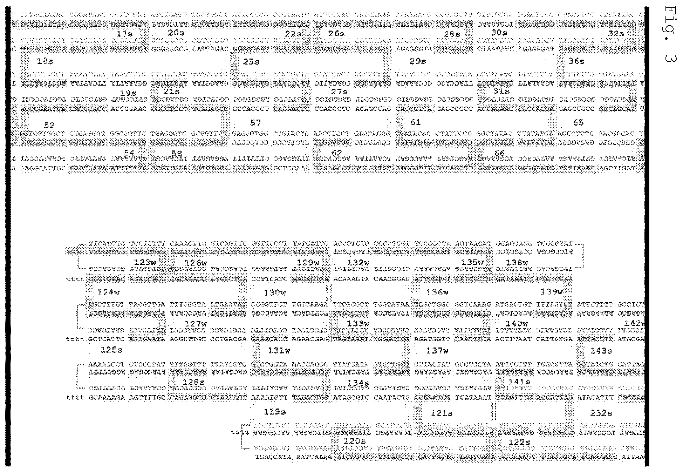

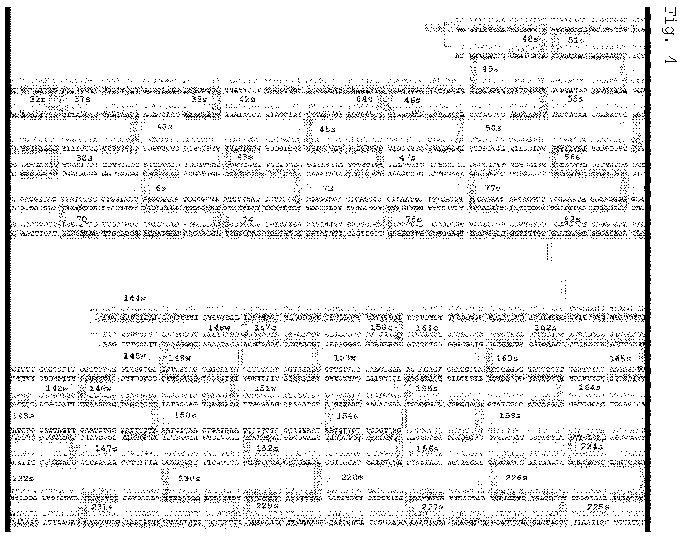

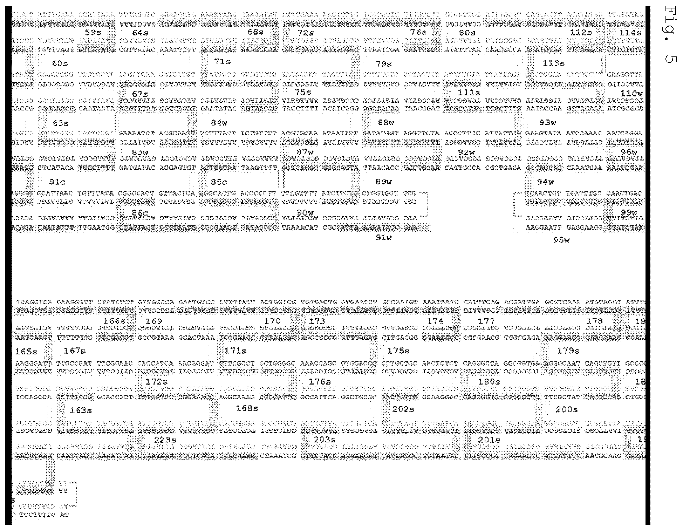

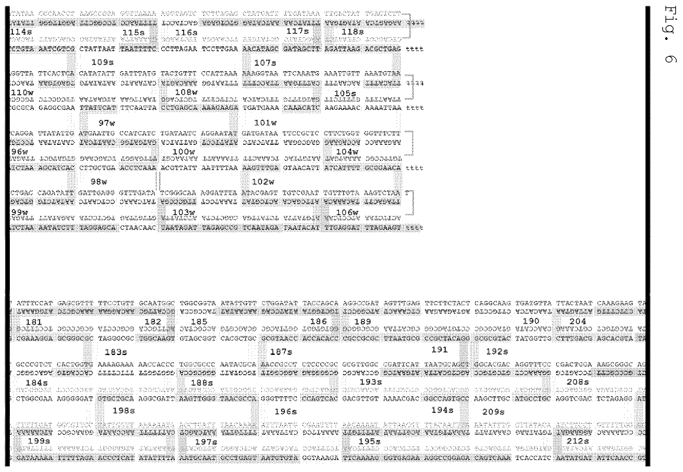

The staple nucleic acid that can form the nucleic acid structure is not particularly limited as long as it contains a sequence complementary to the single-stranded circular nucleic acid, and can fold the single-stranded circular nucleic acid by complementary base pairing with the single-stranded circular nucleic acid to form DNA origami. The number of bases in the staple nucleic acid is not particularly limited, and is, for example, 10 to 500, preferably 20 to 250, and more preferably 30 to 100. In the staple nucleic acid, the number of bases in the sequence complementary to the single-stranded circular nucleic acid is not particularly limited, and is, for example, 10 to 500, preferably 20 to 250, and more preferably 30 to 100. Like the staple DNAs indicated by markers in Figs. 2 to 7, one staple nucleic acid generally contains sequences complementary to a plurality of (e.g., 2 to 5, preferably 2 to 4, and more preferably 2 or 3) adjacent strands that are present when a single-stranded circular nucleic acid is folded. A staple nucleic acid having such sequence characteristics can further stabilize the single-stranded circular nucleic acid in a folded state through complementary base pairing with the single-stranded circular nucleic acid. However, in the nucleic acid structure of the present invention, two staple nucleic acids involved in Holliday junction formation (82s and 162s in Reference Example 1, provided later) exceptionally, rather than forming a complementary base pair across a plurality of adjacent strands that are present when the single-stranded circular nucleic acid is folded, preferably form a complementary base pair with only one of these strands. Various DNA origami structures have been reported so far, and the sequence of staple nucleic acid for forming a nucleic acid structure of the desired shape and configuration can be designed according to known methods and information.

The size of the nucleic acid structure is not particularly limited, and can be suitably adjusted depending on the shape, size, etc. of the test substance. The nucleic acid structure is preferably nano-sized (nano-structure). For example, the longest diameter (major diameter) of the nucleic acid structure is 1000 nm or less. In an embodiment of the present invention, the major diameter of the nucleic acid structure is, for example, 5 to 1000 nm, preferably 20 to 800 nm, more preferably 50 to 600 nm, and even more preferably 100 to 400 nm.

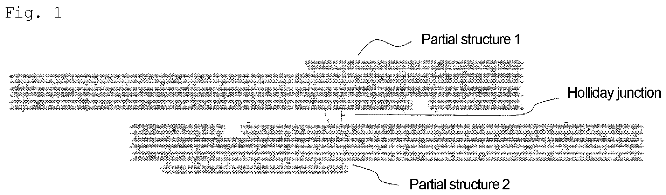

The nucleic acid structure contains a partial nucleic acid structure 1 and a partial nucleic acid structure 2. The partial nucleic acid structure 1 and the partial nucleic acid structure 2 are each part of the above nucleic acid structure, and linked to each other by a Holliday junction (e.g., Fig. 1). That is, the partial nucleic acid structure 1 has two of four arms that form the Holliday junction, and the partial nucleic acid structure 2 has the other two arms. In Reference Example 1, provided later, in the partial nucleic acid structure 1, double-stranded DNA formed by staple DNA with staple number 82s and a single-stranded circular nucleic acid forms two arms, and in the partial nucleic acid structure 2, double-stranded DNA formed by staple DNA with staple number 162s and a single-stranded circular nucleic acid forms the other two arms.

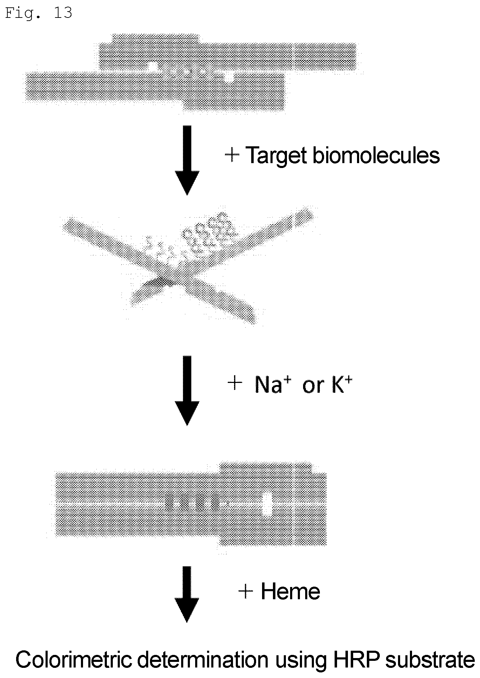

The nucleic acid structure can take three structures (structural type 1, structural type 2, and structural type 3) based on the conformation of the Holliday junction. The structural type 2 is an intermediate structure that mediates structural changes between the structural type 1 and the structural type 3. That is, the structural type 1 can be structurally changed to the structural type 3 via the structural type 2, and the structural type 3 can be structurally changed to the structural type 1 via the structural type 2 (e.g., Fig. 13). As in the Reference Examples and Examples provided later, when the structural type 1 and the structural type 3 differ in the shape of the nucleic acid structure, as an embodiment, the structural type 1 can be expressed as antiparallel type (or parallel type), and the structural type 3 can be expressed as parallel type (or antiparallel type). The structural type 2 can be expressed as cross type because the partial nucleic acid structure 1 and the partial nucleic acid structure 2 are generally fixed in a crossed state.

The shape of the partial structure 1 and partial structure 2 is not particularly limited. Examples of the shape include rectangle, triangle, polygon, ellipse, fan, polyhedron, cone, column, ellipse, etc., or a combination of these shapes. In an embodiment of the present invention, the shape is preferably elongated (e.g., the longest diameter is, for example, 1.5 times or more, 2 times or more, or 4 times or more the shortest diameter (excluding the thickness)).

The size of the partial structure 1 and partial structure 2 is not particularly limited, and can be suitably adjusted depending on the shape, size, etc. of the test substance. The partial structure 1 and partial structure 2 are each preferably nano-sized (nano-structure). For example, the longest diameter (major diameter) of each of the partial structure 1 and partial structure 2 is 1000 nm or less. In an embodiment of the present invention, the major diameter of each of the partial structure 1 and partial structure 2 is, for example, 5 to 1000 nm, preferably 20 to 800 nm, more preferably 50 to 600 nm, and even more preferably 100 to 400 nm.

The partial nucleic acid structure 1 contains a protruding structure 1a containing a nucleic acid aptamer sequence, and the partial nucleic acid structure 2 contains a protruding structure 2a containing a sequence complementary to part or all of the nucleic acid aptamer sequence.

The nucleic acid aptamer sequence is not particularly limited as long as it is a nucleic acid sequence that can bind (preferably specifically bind) to a substance (recognition substance). The recognition substance is not particularly limited as long as it can bind to the nucleic acid aptamer, and examples include nucleic acids, proteins, peptides, low-molecular-weight compounds, physiologically active substances, metal ions, and the like. In an embodiment of the present invention, the recognition substance is preferably a biological material. When the recognition substance is a nucleic acid, the nucleic acid aptamer sequence contains a sequence complementary to the nucleic acid, which is a recognition substance. The nucleic acid aptamer sequence can be a nucleic acid aptamer sequence known for a substance, or a sequence obtained by screening using a known method (e.g., SELEX method). The number of bases in the nucleic acid aptamer sequence is not particularly limited, and is, for example, 5 to 500, preferably 10 to 250, and more preferably 20 to 100.

The sequence complementary to part or all of the nucleic acid aptamer sequence is not particularly limited, but is preferably a sequence complementary to part of the nucleic acid aptamer sequence (e.g., 70% or less, 60% or less, or 50% or less of the nucleic acid aptamer sequence).

The protruding structure 1a and the protruding structure 2a are not particularly limited as long as they contain a nucleic acid aptamer sequence or a sequence complementary to part or all of the nucleic acid aptamer sequence, and protrude in the partial nucleic acid structure 1 and partial nucleic acid structure 2 (e.g., not incorporated into the structure due to complementary pairing etc.). For example, the protruding structure 1a and protruding structure 2a are part of the staple nucleic acid. In this example, the staple nucleic acid contains a sequence complementary to the single-stranded circular nucleic acid, and a nucleic acid aptamer sequence or a sequence complementary to part or all of the nucleic acid aptamer sequence, and the latter forms a protruding structure consisting of a single-stranded nucleic acid without complementarily binding to the single-stranded circular nucleic acid.

The number of protruding structures 1a is not particularly limited, and is, for example, 1 to 30, 2 to 20, 3 to 10, or 2 to 7. The number of protruding structures 2a is not particularly limited, and is, for example, 1 to 30, 2 to 20, 3 to 10, or 2 to 7.

The partial nucleic acid structure 1 contains a protruding structure 1b containing a split domain 1 of an enzyme, and the partial nucleic acid structure 2 contains a protruding structure 2b containing a split domain 2 paired with the split domain 1.

The enzyme used in the present invention is not particularly limited as long as it is a nucleic acid or protein that itself has enzymatic activity or that can exhibit enzymatic activity by binding to other substances, such as cofactors, and as long as it can be split into two (i.e., even if split, it can reassociate and exhibit enzymatic activity). The enzyme is preferably a nucleic acid enzyme containing two or more G-quartets, a photoprotein, or the like. The G-quartet is a planar structure formed by four guanine bases through Hoogsteen hydrogen bonding. Two or more of these are stacked to form a higher-order structure known as a guanine quadruplex. The higher-order structure can exhibit peroxidase activity in the presence of heme. Examples of photoproteins include NanoLuc (Promega). NanoLuc is a luciferase with furimazine as a substrate, and its luminescence intensity is very strong, about 100 times the luminescence intensity of firefly luciferase. LgBiT (Promega) and SmBiT (Promega) are subunits of NanoLuc, and they are reconstituted as a luciferase by interacting with each other. LgBiT is a large subunit of approximately 18 kDa, and SmBiT is a small subunit of 11 amino acid residues. LgBiT and SmBiT have a dissociation constant of 190 µM and extremely low affinity, and they do not express luciferase activity when simply mixed (Andrew, D., (2016) ACS Chem. Biol. 11, 400-408).

The split domain 1 and the split domain 2 are obtained by dividing an enzyme into two. The split position is not particularly limited as long as these domains can reassociate and exhibit enzymatic activity. When the enzyme is a nucleic acid enzyme containing two or more G-quartets, the split domain 1 (split nucleic acid sequence 1) and split domain 2 (split nucleic acid sequence 2) are not particularly limited as long as they are nucleic acid sequences that can associate to form G-quartets in the presence of a monovalent cation (e.g., potassium ion or sodium ion). Examples of such nucleic acid sequences include nucleic acid sequences in which a structural unit consisting of two or more (e.g., 2 to 4, or 2 or 3) consecutive guanine bases and a structural unit consisting of one or more (e.g., 1 to 5, or 2 or 3) consecutive non-guanine bases are alternately arranged. Typical examples of such nucleic acid sequences include telomere element sequences. When the enzyme is a photoprotein such as NanoLuc, the split domain 1 (split peptide sequence 1) and split domain 2 (split peptide sequence 2) are not particularly limited as long as they are peptide sequences that can express luciferase activity under conditions that allow both domains to remain stable in close proximity. The split peptide sequences can be determined pursuant to or according to known information, depending on the type of photoprotein, the desired dissociation constant, etc.

The protruding structure 1b and the protruding structure 2b are not particularly limited as long as they contain a split domain 1 or a split domain 2, and protrude in the partial nucleic acid structure 1 and the partial nucleic acid structure 2 (e.g., not incorporated into the structure due to complementary pairing etc.). For example, the protruding structure 1b and the protruding structure 2b are part of the staple nucleic acid. In this example, the staple nucleic acid contains a sequence complementary to the single-stranded circular nucleic acid, and the split domain 1 or split domain 2, and the latter forms a protruding structure consisting of a single-stranded nucleic acid without complementarily binding to the single-stranded circular nucleic acid.

The number of protruding structures 1b is not particularly limited, and is, for example, 1 to 30, 2 to 20, 3 to 10, or 2 to 7. The number of protruding structures 2b is not particularly limited, and is, for example, 1 to 30, 2 to 20, 3 to 10, or 2 to 7.

The arrangement of the protruding structure 1a and protruding structure 2a is not particularly limited as long as the protruding structure 1a and protruding structure 2a are arranged to face each other and bind together based on complementary base pairing in the absence of a recognition substance for the nucleic acid aptamer sequence. For example, in the case of taking the structural type 1, the protruding structure 1a and the protruding structure 2a are arranged to face each other. When the protruding structure 1a and the protruding structure 2a face each other, it is preferable that the protruding structure 1b and the protruding structure 2b do not face each other.

The arrangement of the protruding structure 1b and protruding structure 2b is not particularly limited as long as the protruding structure 1b and the protruding structure 2b are arranged to face each other to form a nucleic acid enzyme in the presence of a recognition substance for the nucleic acid aptamer sequence (when the nucleic acid enzyme contains two or more G-quartets, further in the presence of a monovalent cation). For example, in the case of taking the structural type 3, the protruding structure 1b and the protruding structure 2b are arranged to face each other. When the protruding structure 1b and the protruding structure 2b face each other, it is preferable that the protruding structure 1a and the protruding structure 2a do not face each other.

The protruding structure 1a, protruding structure 2a, protruding structure 1b, and protruding structure 2b are preferably arranged near the Holliday junction, which is the fulcrum. For example, these protruding structures are preferably arranged, for example, within 300 bases, within 200 bases, or within 150 bases, from the boundary between the two arms that form the Holliday junction in the partial nucleic acid structure 1 or partial nucleic acid structure 2. This makes it possible to keep the target structure more stable in the absence or presence of a test substance, and thus further increase the detection sensitivity of the test substance.

The nucleic acid structure of the present invention can be produced by or according to a known method. For example, the nucleic acid structure of the present invention can be produced by heating a solution containing a single-stranded circular nucleic acid and a staple nucleic acid containing a sequence complementary to the single-stranded circular nucleic acid to a high temperature (e.g., 80°C or higher, 85°C or higher, or 90°C or higher) where base pairing hardly occurs, followed by gradual cooling (so that the temperature drops by 1°C, for example, for 15 seconds to 5 minutes, 30 seconds to 3 minutes, or 45 seconds to 2 minutes).

### 3. Use

As an example is shown in Fig. 13, in the nucleic acid structure of the present invention, in the absence of a test substance, the protruding structure 1a and the protruding structure 2a face each other and bind together based on complementary base pairing, and the structural type 1 (or structural type 3) becomes dominant. On the other hand, in the presence of a test substance, the bond between the protruding structure 1a and the protruding structure 2a is dissociated by the binding of the protruding structure 1a to the test substance. Along with this, the protruding structure 1b and the protruding structure 2b face each other to form a nucleic acid enzyme, and the structural type 3 (or structural type 1) becomes dominant. Accordingly, the test substance can be detected by detecting the activity of the nucleic acid enzyme. Therefore, the nucleic acid structure of the present invention can be used for detecting a test substance (e.g., as a constituent of a test substance detection composition).

From this point of view, the present invention, in an embodiment, relates to a test substance detection composition (the composition of the present invention) comprising the nucleic acid structure of the present invention, and a method for detecting a test substance, comprising bringing the nucleic acid structure of the present invention into contact with a sample that may contain the test substance (the detection method of the present invention). These are described below.

The test substance is not particularly limited as long as it can bind to a nucleic acid aptamer. Examples include nucleic acids, proteins, peptides, low-molecular-weight compounds, physiologically active substances, metal ions, and the like. In an embodiment of the present invention, the test substance is preferably a biological material.

When the test substance is a biological material, in an embodiment thereof, organisms containing the biological material can be detected. Examples of such organisms include viruses. Examples of viruses include enveloped viruses (viruses with envelopes), such as influenza viruses (e.g., type A and type B), rubella virus, Ebola virus, coronaviruses, measles virus, varicella-zoster virus, mumps virus, arbovirus, RS virus, SARS virus, hepatitis viruses (e.g., hepatitis B virus and hepatitis C virus), yellow fever virus, AIDS virus, rabies virus, hantavirus, dengue virus, Nipah virus, and Lyssa virus; non-enveloped viruses (viruses without envelopes), such as adenovirus, norovirus, rotavirus, human papillomavirus, poliovirus, enterovirus, coxsackievirus, human parvovirus, encephalomyocarditis virus, poliovirus, and rhinovirus; and the like. Particularly preferred among these are coronaviruses.

Coronaviruses belong to the subfamily *Orthocoronavirinae.* Examples of coronaviruses include alphacoronaviruses, betacoronaviruses, gammacoronaviruses, deltacoronaviruses, and the like. Preferred among these are betacoronaviruses. Examples of betacoronaviruses include SARS-associated coronavirus (SARSr-CoV), human coronavirus HKU1, MERS coronavirus, and the like. Preferred among these is SARSr-CoV. Examples of SARSr-CoV include SARS-CoV-2, SARS-CoV-1, and the like. Preferred among these is SARS-CoV-2. The agent of the present invention can be preferably used particularly against SARS-CoV-2.

The composition of the present invention may contain other components, if necessary. Examples of other components include bases, carriers, solvents, dispersants, emulsifiers, buffers, stabilizers, excipients, binders, disintegrants, lubricants, thickeners, humectants, colorants, fragrances, chelating agents, and the like.

The composition of the present invention may constitute a kit. The kit may include instruments, reagents, etc. for use in the implementation of the test method of the present invention. Examples of instruments include test tubes, microtiter plates, agarose particles, purification columns, epoxy-coated glass slides, gold colloid-coated glass slides, microtubes, and the like. Examples of reagents include cofactors of the nucleic acid enzyme, monovalent cation-containing buffers, standard samples (positive control and negative control), and the like.

The sample that may contain a test substance used in the detection method of the present invention is not particularly limited. Examples include biological samples. Specific examples include body fluids, such as whole blood, serum, plasma, follicular fluid, menstrual blood, saliva, cerebrospinal fluid, synovial fluid, urine, interstitial fluid, sweat, tears, and saliva; samples derived from these body fluids (e.g., purified products), tissue pieces, samples derived from tissue pieces (e.g., crushed products and purified products), and the like.

Before, during, or after contact of the nucleic acid structure of the present invention with the sample, a monovalent cation can be added, if necessary. For example, when the nucleic acid enzyme contains two or more G-quartets, G-quartets are formed by the presence of a monovalent cation.

Before, during, or after contact of the nucleic acid structure of the present invention with the sample, a cofactor of the nucleic acid enzyme can be added, if necessary. For example, when the nucleic acid enzyme contains two or more G-quartets, peroxidase activity is exerted by the presence of heme, such as hemin.

In an embodiment of the present invention, contact is performed in a solution, but is not limited thereto. The concentration of the nucleic acid structure of the present invention in the solution is not particularly limited, and is, for example, 0.1 to 100 nM, preferably 0.5 to 20 nM, and more preferably 1.5 to 10 nM. When the solution contains a monovalent cation, the concentration thereof is, for example, 10 mM to 1 M, preferably 50 to 600 mM, and more preferably 100 to 300 mM.

The pH of the solution in which contact is performed is preferably 2 to 10, more preferably 3.5 to 7.5, and even more preferably 4.5 to 5.5, in terms of allowing detection in a shorter time.

After contact, the enzymatic activity of the nucleic acid enzyme can be detected to detect the test substance. The enzymatic activity can be detected by or according to a known method. In a preferred embodiment of the present invention, it is preferable to add a substrate that becomes colored due to the enzymatic activity of the nucleic acid enzyme. As a result, the presence or absence of color change (i.e., the presence or absence and amount of test substance) can be detected or measured by visual observation or simple absorbance measurement. When the enzymatic activity is peroxidase activity, examples of such substrates include TMB (3,3',5,5'-tetramethylbenzidine), OPD (o-phenylenediamine dihydrochloride), ABTS (2,2'-azinobis[3-ethylbenzothiazoline-6-sulfonic acid]-diammonium salt), and the like.

### Examples

The present invention is described in detail below based on Examples; however, the present invention is not limited by these Examples.

### Reference Example 1: Preparation 1 of Nucleic Acid Structure

As single-stranded circular DNA, M13mp18 (SEQ ID NO: 273) was purchased and prepared from tilibit nanosystems. Further, staple DNAs (SEQ ID NOs: 1 to 236) constituting a nucleic acid structure by complementary binding to the single-stranded circular DNA were purchased and prepared from IDT. M13mp18 and each staple DNA were mixed in a solution containing 40 mM of Tris, 20 mM of acetic acid, 2 mM of EDTA, and 12.5 mM of magnesium acetate (1×TAE/Mg²⁺ solution) so that the final concentration of M13mp18 was 4 nM and the final concentration of each staple DNA was 20 nM. The mixed solution was heated to 90°C by a PCR thermal cycler and then cooled to 25°C at a rate of - 1.0°C per minute. Excess staple was removed using a centrifugal ultrafiltration filter (Amicon Ultra 0.5 mL-100 K, Millipore).

Atomic force microscope (AFM) observation was performed, and it was confirmed that a pincher- or plier-like nucleic acid structure was formed. The major diameter was around 200 nm.

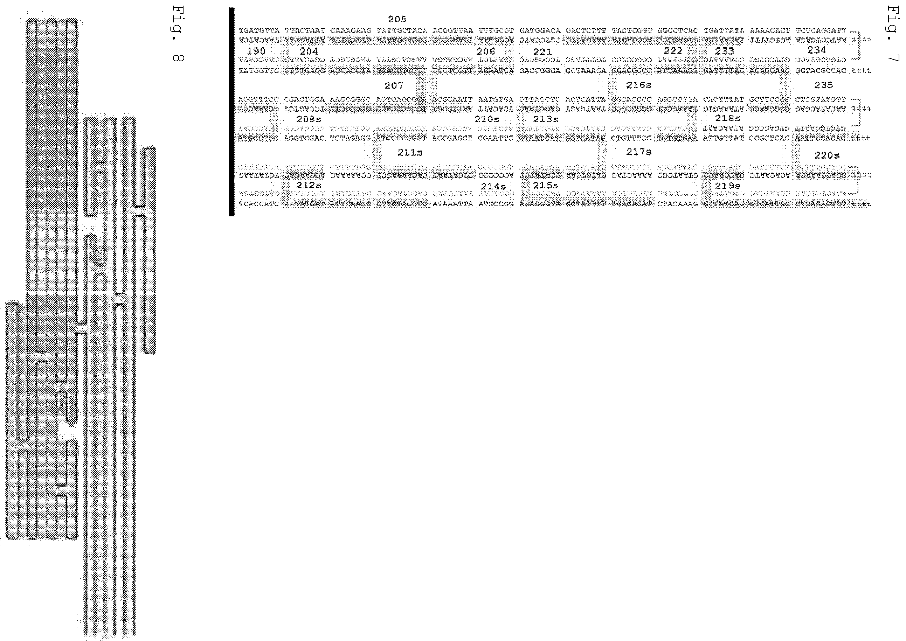

Tables 1 to 6 show the staple numbers, base sequences, and SEQ ID Nos of the staple DNAs used. Fig. 1 shows the overall view of the nucleic acid structure, and Figs. 2 to 7 show diagrams obtained by dividing the overall view into six parts. Further, Fig. 8 shows a schematic diagram showing only single-stranded circular DNA in a linear form in the nucleic acid structure.

**Table 1**

| staple No. | Sequence | SEQ ID No |
|---|---|---|
| 1s | CCTAATTTTTTTATCCTGAATCTTACCAACGCTATTTT | 1 |
| 2s | TTTTACGAGCGTCTGCAGCACCGTTTTT | 2 |
| 3s | CAAGTTTGAATGAAACCATCGATATTCCAGAG | 3 |
| 4s | AGCTACAAGCCAGTTACAAAATAAGGCCGGAA | 4 |
| 5s | ACGTCACCCCTTTAGCGTCAGACTACAACGCC | 5 |
| 6s | ATTATTTAAAGATTAGTTGCTATTTTGCACCC | 6 |
| 7s | TTTTCATCCCATTACCATTAGCAAACAGCCAT | 7 |
| 8s | TTGAAGCCTTAAATCTCCCAAT | 8 |
| 9s | CCAAATAAGAAACGATTTAGAGCCAGCA | 9 |
| 10 | TTTTAATCAGTAGCATAGTTAGCGTTTT | 10 |
| 11 | TCGTCTTTTCCACAGACAGCCCTCGACAGAAT | 11 |
| 12 | TTTTTAACGATCTAAAGTTTTG | 12 |
| 13s | AAATCACCAGTAGCAGGCATTT | 13 |
| 14s | TGCCATCTGAGCCATTTGGGAATTTTTGTTTA | 14 |
| 15s | GCGTTTTAAATGAAAATAGCAGCCTCACCGTC | 15 |
| 16s | ACCGACTTTTTCATAATCAAAATCCTCATTTT | 16 |
| 17s | TAAAAACAATAGAAGGCTTATCCGGTATTCTAAGAACGCGAG | 17 |
| 18s | AGGTGAATTATTTACAGAGAGAATAACA | 18 |
| 19s | ACCGGAACGGAAATTATTCATTAA | 19 |
| 20s | AATCAGATGGGAAGCGCATTAGACGAAGGTAA | 20 |
| 21s | ATATTGACCGCCTCCCTCAGAGCCAGAACCGC | 21 |
| 22s | TAACTGAACATTACCGCGCCCAATAGCAAGCA | 22 |
| 23 | TGTATGGGGTCACCAGTACAAACTGTAGCGCG | 23 |
| 24 | TGTAGCATCCAGACGTTAGTAAATGAATTTTC | 24 |
| 25s | CAGAACCGAACCGATTGAGGGAGGGGGAGAAT | 25 |
| 26s | GTAGGAATCACCCTGAACAAAGTCCAAAAGGG | 26 |
| 27s | CGACATTCCCACCCTCAGAGCCACCCGTACTC | 27 |
| 28s | ATTGAGCGAAGCAAGCCGTTTTTATTTTCATC | 28 |
| 29s | GAGCCGCCTTACCAGCGCCAAAGAAGAGGGTA | 29 |
| 30s | TCGAGAACCTAATATCAGAGAGATAGAAAATT | 30 |
| 31s | CATATGGTACCAGAACCACCACCATGATATAA | 31 |
| 32s | AGAATTGAGTATTAAACCAAGTACCGCACTCA | 32 |
| 33 | TCGGTCATGTAACACTGAGTTTCATTTTGC | 33 |
| 34 | CCCATGTACCAGCCCCCTTATTAGCGTT | 34 |
| 35 | TAAACAACTTTCAACAGTTTCAGCGG | 35 |
| 36s | GCCAGCATTTTTGTCACAATCAATAACCCACA | 36 |
| 37s | AAGAACGGGTTAAGCCCAATAATACCACGGAA | 37 |
| 38s | TAAGTTTATGACAGGAGGTTGAGGAGTACCAG | 38 |
| 39s | AAACAATGTCGGCTGTCTTTCCTTATCATTCC | 39 |
| 40s | ACGATTGGAAGAAACGCAAAGACAAGAGCAAG | 40 |

**Table 2**

| staple No. | Sequence | SEQ ID No |
|---|---|---|
| 41 | AGTGAGAAGCAAGCCCAATAGGAA | 41 |
| 42s | ATCAATAAAAATAGCAATAGCTATAGGTGGCA | 42 |
| 43s | ACATATAACCTTGATATTCACAAAAGAGAAGG | 43 |
| 44s | AGCCCTTTTAATTTACGAGCATGTAGAAACCA | 44 |
| 45s | TCCTCATTGAAAATACATACATAACTTACCGA | 45 |
| 46s | AAATAATATCCCATCCTTAAGAAAAGTAAGCATATGTTAG | 46 |
| 47s | CAAACGTAAAAGCCAGAATGGAAAACATGAAA | 47 |
| 48s | GAATCATAATAAGGCGTTAAATAAGA | 48 |
| 49s | AACAAAGTAAGTCCTGAACAAGAAAAACACCG | 49 |
| 50s | TCTGAATTACTCCTTATTACGCAGGATAGCCG | 50 |
| 51s | TGTGATAAATTACTAGAAAAAGCCTTTATCAA | 51 |
| 52 | AGCCACCACCACCGGAACCAGAGCCACC | 52 |
| 53 | CAGGGATATAGAAAGGAACAACTAAAGGAATTGC | 53 |
| 54 | GAATAATAATTTTTTCGAACCGCCACCCTCAG | 54 |
| 55s | CAATAGATTACCAGAAGGAAACCGAACTGGCA | 55 |
| 56s | TGATTAAGTACCGTTCCAGTAAGCCCCCTGCC | 56 |
| 57 | GCTCCAAATAGTACCGCCACCCTCGCCACCCT | 57 |
| 58 | CACCCTCAACGTTGAAAATCTCCAAAAAAAAG | 58 |
| 59s | ATCATATGTTTAATGGTTTGAAATACCGACCG | 59 |
| 60s | CAATAATAATGCAGAACGCGCCTGTGTTTAGT | 60 |
| 61 | ATCAGCTTCGGAATAGGTGTATCACACCCTCA | 61 |
| 62 | AGGAGGTTAGGAGCCTTTAATTGTATCGGTTT | 62 |
| 63s | TGGCTTTTACGGAATACCCAAAAGAGGAAACG | 63 |
| 64s | GACCTAAACGTTATACAAATTCTTACAACATG | 64 |
| 65 | AGCTTGATGTGCCGTCGAGAGGGTGAGCCGCC | 65 |
| 66 | GTATAGCCGCTTTCGAGGTGAATTTCTTAAAC | 66 |
| 67s | TTCAGCTAAGGTTTAACGTCAGATAATTGCGT | 67 |
| 68s | AAAGCCAAATATTTTAGTTAATTTCATCTTCT | 68 |
| 69 | AACAACCATAGCGGGGTTTTGCTCCAGGTCAG | 69 |
| 70 | GCGGATAAACCGATAGTTGCGCCGACAATGAC | 70 |
| 71s | AGTAACAGCAGACGACGACAATAAACCAGTAT | 71 |
| 72s | TTTCAAATCGCTCAACAGTAGGGCGTAAAGTA | 72 |
| 73 | CGGTCGCTAGGCTGAGACTCCTCACAAATAAA | 73 |
| 74 | ATTAGGATTCGCCCACGCATAACCGATATATT | 74 |
| 75s | ATTCTGTCTACCTTTTACATCGGGAAAATTAT | 75 |
| 76s | GAATCGCCAAGACAAAGAACGCGAGAAAACTT | 76 |
| 77s | TATTTCGGAACCTATTATTCTGAAGCGCAGTC | 77 |
| 78s | GTATTAAGGAGGCTTGCAGGGAGT | 78 |
| 79s | TAACGGATAAAGTACCGACAAAAGTTAATTGA | 79 |
| 80s | CCAATCGCATATTTAACAACGCCAAGTAATAA | 80 |

**Table 3**

| staple No. | Sequence | SEQ ID No |
|---|---|---|
| 81c | TTGAATGGTATAAACAGTTAATGCGTCATACA | 81 |
| 82s | TAAAGGCCGCTTTTGCGAATACGTGGCACAGACAATATTT | 82 |
| 83w | AGATTTTCGATGATACAGGAGTGTTGAGTAAC | 83 |
| 84w | TAAGTTTTAAAACAGAAATAAAGAGAATATAC | 84 |
| 85c | GATAGCCCAACGGGGTCAGTGCCTACTGGTAA | 85 |
| 86c | AGTGCCCGCTATTAGTCTTTAATGCGCGAACT | 86 |
| 87w | TTGCACGTGGTGAGGCGGTCAGTACAGAAGAT | 87 |
| 88w | GCCTGCAATAGAACCTACCATATCAGAAACAA | 88 |
| 89w | CGAACCACCAGTTAACACC | 89 |
| 90w | AAAACAGATAAAACATCGC | 90 |
| 91w | CATTAAAAATACCGAA | 91 |
| 92w | GGAAGGGTCAGTGCCACGCTGAGA | 92 |
| 93w | CAAATGAAGTTTGGATTATACTTCAATACCAA | 93 |
| 94w | GCAAATCAACAGTTGAGCCAGCAG | 94 |
| 95w | AAGGAATTGAGGAAGG | 95 |
| 96w | TCCTGATTAAATCTAAAGCATCACAATATCTG | 96 |
| 97w | ACCTCAAAGATGATGGCAATTCATTTATTCAT | 97 |
| 98w | CTAACAACTATCAAACCCTCAATCCTTGCTGA | 98 |
| 99w | GTCAGTTGTTATCTAAAATATCTTTAGGAGCA | 99 |
| 100w | GATTATCAACGTTATTAATTTTAATAAATCCT | 100 |
| 101w | GTAACATTGAGCGGAATTATCATCTGATGAAA | 101 |
| 102w | TAATACATATTCGACAACTCGTATAAGTTTGA | 102 |
| 103w | TTGCCCGATAATAGATTAGAGCCGTCAATAGA | 103 |
| 104w | ACCAGAAGATCATTTTGCGGAACATTTTTTAGACTT | 104 |
| 105s | AACAATTTAAGAAAACAAAATTAATTTTAAGAAACC | 105 |
| 106w | TACAAACATTGAGGATTTAGAAGTTTTT | 106 |
| 107s | CAAACATCCATTTGAATTACCTTTAACATAGC | 107 |
| 108w | AAACAGTACCTGAGCAAAAGAAGAATATTCCT | 108 |
| 109s | TTCAATTACATAAATCAATATATGCTATTAAT | 109 |
| 110w | TAACCTTGATCGCGCAGAGGCGAACAATATAA | 110 |
| 111s | GAGAATATTCGCCTGATTGCTTTGTGAATAAT | 111 |
| 112s | TTTAGGCACTATATGTAAATGCTGATGCAAAT | 112 |
| 113s | GTTACAAAGAGGCATTTTCGAGCCACATGTAA | 113 |
| 114s | TTATATAACTTCTGTAAATCGTCGTGAGTGAA | 114 |
| 115s | TAATTTTCTTTTAACCTCCGGCTTAGGTTGGG | 115 |
| 116s | GACTACCTCCTTAGAATCCTTGAATTTAATGG | 116 |
| 117s | GATAGCTTTTTATCAAAATCATAGGTCTGAGA | 117 |
| 118s | GAGTCAATAGTGAAAGATTAAGACGCTGAGTTTTTTACATTT | 118 |
| 119s | ACCATAAATCAAAAGTTCAGAAAACGAGAATTTTAAAATGTT | 119 |
| 120s | TTTAAACAATCAGGTCTTTACCCTGACTATTA | 120 |

**Table 4**

| staple No. | Sequence | SEQ ID No |
|---|---|---|
| 121s | TAGTCAGAATTCATTGAATCCCCCCGGAATCG | 121 |
| 122s | GAGTAGATAGCAAAGCGGATTGCATCAAAAAG | 122 |
| 123w | AGACCAGGAAAGAGGACAGATGAATTTT | 123 |
| 124w | AGTGAATATCAACGTAACAAAGCTTTTTCGGTGTAC | 124 |
| 125s | AAAAGAAGTTTTGCATAGCGAGAGGCTTTTTTTTGCTCATTC | 125 |
| 126w | CAACTTTGCGCATAGGCTGGCTGAATATTCAT | 126 |
| 127w | TACCCAAAAGGCTTGCCCTGACGAGACGATAA | 127 |
| 128s | AAACCAAACAGAGGGGGTAATAGT | 128 |
| 129w | AAGAGTAACAATCATAAGGGAACCGAACTGAC | 129 |
| 130w | AGAACGAGTCTTGACAAGAACCGGCCTTCATC | 130 |
| 131w | TAGACTGGCCCTCGTTTACCAGACGAAACACC | 131 |
| 132w | CAGACGGTACAAAGTACAACGGAGTTATACCA | 132 |
| 133w | AGCGCGAATAGTAAATTGGGCTTGAGCAACAC | 133 |
| 134s | TATCATAAATAGCGTCCAATACTGTCAAATGC | 134 |
| 135w | CATCGCCTATGTTACTTAGCCGGAACGAGGCG | 135 |
| 136w | TAATTTCACTTTGACCCCCAGCGAATTTGTAT | 136 |
| 137w | TCATAAATTACGAGGCATAGTAAGAGATGGTT | 137 |
| 138w | ATCCGCGACCTGCTCC | 138 |
| 139w | GATAAATTGTGTCGAAACACTAAA | 139 |
| 140w | ACACTCATACTTTAATCATTGTGATAACGCCA | 140 |
| 141s | AAAGGAATTTAGTTTGACCATTAGCTGCGAAC | 141 |
| 142w | ATGCGATTAAAGAGGCAAAAGAAT | 142 |
| 143s | CGCAAATGAACTAATGCAGATACAATTACCTT | 143 |
| 144w | AGACTTTTTCATGAGG | 144 |
| 145w | AAGTTTCCATTGCACCAAC | 145 |
| 146w | CTAAAACGTTAAGAACTGGCTCATTAGGAATA | 146 |
| 147s | CCACATTCGTCAATAACCTGTTTAGTTTCATT | 147 |
| 148w | AAAATACGTTGAGGACTAA | 148 |
| 149w | TCAGGACGTAATGCCACTACGAAGAAACGGGT | 149 |
| 150s | TTCATTTGTTCATCAGTTGAGATTTATACCAG | 150 |
| 151w | ATTAAAGATTGGGAAGAAAAATCTATTACAGG | 151 |
| 152s | TAGAAAGAGGGCGCGAGCTGAAAATTAAATAT | 152 |
| 153w | AAAACGAATCCAGTTTGGAACAAGCAAAGGGC | 153 |
| 154s | CAATTCTACTAACGGAACAACATTACGTTAAT | 154 |
| 155s | AGTGTTGTTGAGGGGACGACGACACGTGCATC | 155 |
| 156s | TGCCAGTTCTAATAGTAGTAGCATTTGCTGAA | 156 |
| 157c | CAGAGGCTACGTGGACTCCAACGTAGTCCACT | 157 |
| 158c | GAAAAACCTCGGAACGAGGGTAGCAACGGCTA | 158 |
| 159s | AATAAATCATGGGCGCATCGTAACGTATCGGC | 159 |
| 160s | CTCAGGAAAAAGAATAGCCCGAGAGCCCACTA | 160 |

**Table 5**

| staple No. | Sequence | SEQ ID No |
|---|---|---|
| 161c | AGACAGCAGTCTATCAGGGCGATGTAGGGTTG | 161 |
| 162s | TGACCTGAAAGCGTAAGGGATCGTCACCCTCAGCAGCGAA | 162 |
| 163s | GAATTAGCAAAATTAATGACCGTAATGGGATAGCTTTCCG | 163 |
| 164s | ATAAATCAGATCGCACTCCAGCCAGGTCACGT | 164 |
| 165s | CGTGAACCATCACCCAAATCAAGTAATCCCTT | 165 |
| 166s | GTCGAGGTAGAGATAGAACCCTTC | 166 |
| 167s | GCACCGCTGTTCCGAAATCGGCAATTTTTGGG | 167 |
| 168s | CTAAATCGCGTCGGATTCTCCGTGAGGCAAAG | 168 |
| 169 | TGGCCAACGCCGTAAAGCACTAAAATCCTGTT | 169 |
| 170 | CTAAAGGGCGACCAGTAATAAAAGGGACATTC | 170 |
| 171s | CGCCATTCGCCCCAGCAGGCGAAATCGGAACC | 171 |
| 172s | TGATGGTGTCTGGTGCCGGAAACCGGAACAAA | 172 |
| 173 | CAGTCACAAGCCCCCGATTTAGAGCGGTCCAC | 173 |
| 174 | GGAAAGCCGATTATTTACATTGGCAGATTCAC | 174 |
| 175s | GGAAGGGCAGAGAGTTGCAGCAAGCTTGACGG | 175 |
| 176s | GCTGGTTTGCCATTCAGGCTGCGCTGAGCGAG | 176 |
| 177 | CTGAAATGGGCGAACGTGGCGAGATCACCGCC | 177 |
| 178 | GAAGAAAGACCTACATTTTGACGCTCAATCGT | 178 |
| 179s | TACGCCAGAACAGCTGATTGCCCTAAGGAAGG | 179 |
| 180s | TGGCCCTGGATCGGTGCGGGCCTCTTCCTGTA | 180 |
| 181 | ATGGAAATCGAAAGGAGCGGGCGCCACCAGTG | 181 |
| 182 | TGGCAAGTGCCATTGCAACAGGAAAAACGCTC | 182 |
| 183s | AGGCGATTGGGTGGTTTTTCTTTTTAGGGCGC | 183 |
| 184s | AGACGGGCCTGGCGAAAGGGGGATGGAACGCC | 184 |
| 185 | TACCGCCAGTAGCGGTCACGCTGCTGCGTATT | 185 |
| 186 | ACCACACCTGCTGGTAATATCCAGAACAATAT | 186 |
| 187s | CCAGTCACGCGGGGAGAGGCGGTTGCGTAACC | 187 |
| 188s | GGGCGCCAAAGTTGGGTAACGCCATTTTGTTA | 188 |
| 189 | CTCAAACTATCGGCCTCGCCGCGCTTAATGCG | 189 |
| 190 | TATGGTTGTAACATCACTTGCCTGAGTAGAAGAA | 190 |
| 191 | CCGCTACAGGAGCTGCATTAATGAATCG | 191 |
| 192s | ATGCCTGCGGAAACCTGTCGTGCCGCGCGTAC | 192 |
| 193s | GCCAACGCGACGTTGTAAAACGAC | 193 |
| 194s | GGCCAGTGCCTAAATTGTAAACGTTAAT | 194 |
| 195s | ATTTTGTTTTCAAAAGGGTGAGAAAGGCCGGAGACAGTCAAA | 195 |
| 196s | GGTAAAGAAAAATTCGCATTAAATGGGTTTTC | 196 |
| 197s | AATCAGCTAATGCAATGCCTGAGTAATGTGTA | 197 |
| 198s | ATATTTTACATTTTTTAACCAATAGTGCTGCA | 198 |
| 199s | ATCAAAAAGATAAAAATTTTTAGAACCCTCAT | 199 |
| 200s | AACGCAAGTAATTCGCGTCTGGCCTTCGCTAT | 200 |

**Table 6**

| staple No. | Sequence | SEQ ID No |
|---|---|---|
| 201s | GCCAGCTTTTTTGCGGGAGAAGCCTTTATTTC | 201 |
| 202s | TGTAATACTCATCAACATTAAATGAACTGTTG | 202 |
| 203s | TAACAACCGTTGTACCAAAAACATTATGACCC | 203 |
| 204 | ATTAGTAACTTTGACGAGCACGTA | 204 |
| 205 | TTAACCGTTGTAGCAATACTTCTTTG | 205 |
| 206 | AATTGCGTTCCTCGTTAGAATCAACGCAAA | 206 |
| 207 | TAACGTGCTTTGCGCTCACTGCCCGCTT | 207 |
| 208s | TCCAGTCGAGGTCGACTCTAGAGGCCAAAAAC | 208 |
| 209s | TCACCATCTGTATAAGCAAATATTAAGCTTGC | 209 |
| 210s | ACCGAGCTCGAATTCTCACATT | 210 |
| 211s | TTGATAATCAGAAAAGCCATCCCCGGGT | 211 |
| 212s | AGGAAGATAATATGATATTCAACCGTTCTAGCTG | 212 |
| 213s | GAGCTAACGTAATCATGGTCATAGCATGTCAA | 213 |
| 214s | ATAAATTAATGCCGGACCCCGG | 214 |
| 215s | TCATATGTAGAGGGTAGCTATTTTTGAGAGAT | 215 |
| 216s | TGTGTGAATAAAGCCTGGGGTGCCGGAGGCCG | 216 |
| 217s | CTACAAAGGTAATCGTAAAACTAGCTGTTTCC | 217 |
| 218s | ATAAAGTGATTGTTATCCGCTCACAGAGAATC | 218 |
| 219s | GATGAACGGCTATCAGGTCATTGCCTGAGAGTCTTTTT | 219 |
| 220s | TTTTGGAGCAAACAAATTCCACACTTTT | 220 |
| 221 | TGTCCATCGAGCGGGAGCTAAACATAATGAGT | 221 |
| 222 | ATTAAAGGGTGAGGCCACCGAGTAAAAGAGTC | 222 |
| 223s | CGGCGGATGCAATAAAGCCTCAGAGCATAAAG | 223 |
| 224s | TGGTGTAGATACAGGCAAGGCAAAGAGGTCAT | 224 |
| 225s | TTTTGCGGTTAATTGCTCCTTTTG | 225 |
| 226s | GAGTACCTATGGCTTAGAGCTTAATAACATCC | 226 |
| 227s | TATAATGCAAACTCCAACAGGTCAGGATTAGA | 227 |
| 228s | CCGGAAGCTGTAGCTCAACATGTTGGTGGCAT | 228 |
| 229s | GCAACTAAATTCGAGCTTCAAAGCGAACCAGA | 229 |
| 230s | GCGTTTTAAGTACGGTGTCTGGAAGCTATATT | 230 |
| 231s | CCATATAAGAAGCCCGAAAGACTTCAAATATC | 231 |
| 232s | ATTAAGAGCAGTTGATTCCCAATTATACATTT | 232 |
| 233 | TATAATCAGATTTTAGACAGGAACCCGGAAGC | 233 |
| 234 | TTTTAATCCTGAGAAGTGTTTT | 234 |
| 235 | TTTTAACATACGAGGGTACGCCAGTTTT | 235 |
| 8s-2 | ACGTCAAAGCGAACCTCCCGACTTGCGGGAGGTT | 236 |

### Reference Example 2: Preparation 2 of Nucleic Acid Structure

A nucleic acid structure was prepared in the same manner as in Reference Example 1, except that some staple DNAs were changed to other staple DNAs. Table 7 shows the corresponding staple numbers of Reference Example 1, and the staple numbers, base sequences, and SEQ ID Nos of the staple DNAs used. The configuration of the obtained nucleic acid structure was the same as that of the nucleic acid structure of Reference Example 1, except that the partial structure 1 and the partial structure 2 each contained a protruding structure consisting of single-stranded DNA of the following telomere element sequence.

**Table 7**

| Corresponding staple No. of Ref. Ex. 1 | staple No. | Sequence | SEQ ID No |
|---|---|---|---|
| 65 | 65long | TCTTAAACAGCTTGATGTGCCGTCGAGAGGGTGAGCCGCC | 237 |
| 66 | 66shortTelo | GTATAGCCGCTTTCGAGGTGAATTTTTTTAGGGTTAGGGT | 238 |
| 69 | 69long | ACAATGACAACAACCATAGCGGGGTTTTGCTCCAGGTCAG | 239 |
| 70 | 70shortTelo | GCGGATAAACCGATAGTTGCGCCGTTTTTAGGGTTAGGGT | 240 |
| 73 | 73long | GATATATTCGGTCGCTAGGCTGAGACTCCTCACAAATAAA | 241 |
| 74 | 74shortTelo | ATTAGGATTCGCCCACGCATAACCTTTTTAGGGTTAGGGT | 242 |
| 78s | 78s-telo | GTATTAAGGAGGCTTGCAGGGAGTTTTTTAGGGTTAGGGT | 243 |
| 166s | 166s-telo | GTCGAGGTAGAGATAGAACCCTTCTTTTTAGGGTTAGGGT | 244 |
| 169 | 169long | GGACATTCTGGCCAACGCCGTAAAGCACTAAAATCCTGTT | 245 |
| 170 | 170shortTelo | CTAAAGGGCGACCAGTAATAAAAGTTTTTAGGGTTAGGGT | 246 |
| 173 | 173long | AGATTCACCAGTCACAAGCCCCCGATTTAGAGCGGTCCAC | 247 |
| 174 | 174shortTelo | GGAAAGCCGATTATTTACATTGGCTTTTTAGGGTTAGGGT | 248 |
| 177 | 177long | TCAATCGTCTGAAATGGGCGAACGTGGCGAGATCACCGCC | 249 |
| 178 | 178shortTelo | GAAGAAAGACCTACATTTTGACGCTTTTTAGGGTTAGGGT | 250 |

Staple DNA with a staple number labeled "Telo" contains a telomere element sequence (including two G triplet repeats). The telomere element sequence forms a protruding structure consisting of single-stranded DNA without complementary binding to M13mp18. Among the three structures based on the conformation of the Holliday junction (Fig. 9: provided that Cross is an intermediate structure that mediates structural changes between Antiparallel and Parallel), when taking Parallel, the protruding structure in the partial structure 1 and the protruding structure in the partial structure 2 are arranged so that these protruding structures face each other (i.e., staple DNA containing a telomere element sequence is arranged). The telomere element sequence in the partial structure 1 and the telomere element sequence in the partial structure 2 can be linked in the presence of a monovalent cation to form a G-quartet structure (nucleic acid enzyme: heme stacks can exhibit peroxidase (HRP) activity).

A KCl solution (1 M KCl was dissolved in 1×TAE/Mg²⁺ solution) was added, or not added, to a solution containing the prepared nucleic acid structure, followed by still standing for 5 minutes. AFM observation was then performed, and the presence of K⁺ was visualized as a structural difference of the nucleic acid structure. The solution containing the nucleic acid structure contained 3.2 nM of the nucleic acid structure and 0 mM of KCl or 200 mM of KCl. Further, each structure of the nucleic acid structure was counted from the acquired images, and its proportion was examined.

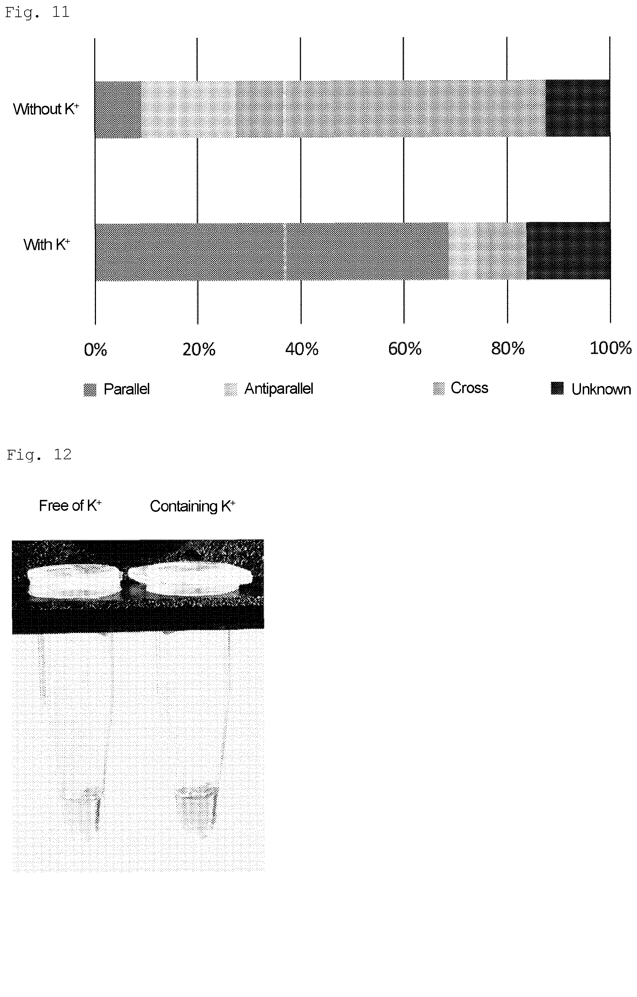

Fig. 10 shows AFM observation images. Further, Fig. 11 shows the proportion of each structure. A structural change to the parallel type due to the addition of K⁺ was confirmed.

### Reference Example 3: Detection 1 of Enzymatic Activity

A KCl solution (1 M KCl was dissolved in 1×TAE/Mg²⁺ solution) was added, or not added, to a solution containing the nucleic acid structure (Reference Example 2), followed by still standing for 5 minutes. Then, hemin (dissolved in DMSO at 100 µM) was added, followed by still standing for 1 hour. The solution containing the nucleic acid structure contained 3.2 nM of the nucleic acid structure, 4.0 µM of hemin, and 0 mM of KCl or 200 mM of KCl. Then, an equivalent amount of 1-Step Ultra TMB-ELISA (catalog number: 34028, Thermo Scientific) was added, and the presence of K⁺ was visualized as a color.

The results are shown in Fig. 12. A strong blue color was developed when a large amount of parallel nucleic acid structure was contained (K⁺ addition). This revealed that enzymatic activity can be exhibited by forming a G-quartet structure in the parallel nucleic acid structure.

### Example 1: Preparation 3 of Nucleic Acid Structure

A nucleic acid structure was prepared in the same manner as in Reference Example 1, except that some staple DNAs were changed to other staple DNAs. Tables 8 and 9 show the corresponding staple numbers of Reference Example 1, and the staple numbers, base sequences, and SEQ ID Nos of the staple DNAs used. The configuration of the obtained nucleic acid structure was the same as that of the nucleic acid structure of Reference Example 1, except that the partial structure 1 and the partial structure 2 each contained a protruding structure consisting of single-stranded DNA of a telomere element sequence, the partial nucleic acid structure 1 contained a protruding structure containing a nucleic acid aptamer sequence (complementary sequence of miR-20 (Mature sequence hsa-miR-20a-5p: SEQ ID NO: 274)), and the partial nucleic acid structure 2 contained a sequence complementary to part of the nucleic acid aptamer sequence.

**Table 8**

| Corresponding staple No. of Ref. Ex. 1 | staple No. | Sequence | SEQ ID No |
|---|---|---|---|
| 65 | 65long | TCTTAAACAGCTTGATGTGCCGTCGAGAGGGTGAGCCGCC | 251 |
| 66 | 66shortTelo | GTATAGCCGCTTTCGAGGTGAATTTTTTTAGGGTTAGGGT | 252 |
| 69 | 69long | ACAATGACAACAACCATAGCGGGGTTTTGCTCCAGGTCAG | 253 |
| 70 | 70shortTelo | GCGGATAAACCGATAGTTGCGCCGTTTTTAGGGTTAGGGT | 254 |
| 73 | 73LongCompM20 | GTGCAGGTAGTTTTGATATATTCGGTCGCTAGGCTGAGAC TCCTCACAAATAAA | 255 |
| 74 | 74shortTelo | ATTAGGATTCGCCCACGCATAACCTTTTTAGGGTTAGGGT | 256 |
| 78s | 78s-telo | GTATTAAGGAGGCTTGCAGGGAGTTTTTTAGGGTTAGGGT | 257 |
| 166s | 166s-telo | GTCGAGGTAGAGATAGAACCCTTCTTTTTAGGGTTAGGGT | 258 |
| 169 | 169LongCompM20 | GTGCAGGTAGTTTTGGAACATTTCTGGCCAACGCCGTAAA GCACTAAAATCCTGTT | 259 |
| 170 | 170shortTelo | CTAAAGGGCGACCAGTAATAAAAGTTTTTAGGGTTAGGGT | 260 |
| 173 | 173long | AGATTCACCAGTCACAAGCCCCCGATTTAGAGCGGTCCAC | 261 |
| 174 | 174shortTelo | GGAAAGCCGATTATTTACATTGGCTTTTTAGGGTTAGGGT | 262 |
| 177 | 177long | TCAATCGTCTGAAATGGGCGAACGTGGCGAGATCACCGCC | 263 |
| 178 | 178shortTelo | GAAGAAAGACCTACATTTTGACGCTTTTTAGGGTTAGGGT | 264 |

**Table 9**

| Corresponding staple No. of Ref. Ex. 1 | staple No. | Sequence | SEQ ID No |
|---|---|---|---|
| 81c | 81cLong | CAATATTTTTGAATGGTATAAACAGTTAATGCGTCATACA | 265 |
| 82s | 82sBothLongM20 | GTGCAGGTAGTTTTTAAAGGCCGCTTTTGCGAATACGTGG CACAGATTTTCTACCTGCACTATAAGCACTTTA | 266 |
| 85c | 85cLong | CGCGAACTGATAGCCCAACGGGGTCAGTGCCTACTGGTAA | 267 |
| 86c | 86cShortMainM20 | AGTGCCCGCTATTAGTCTTTAATGTTTTCTACCTGCACTAT AAGCACTTTA | 268 |
| 157c | 157cLong | AAACGGCTACAGAGGCTACGTGGACTCCAACGTAGTCCACT | 269 |
| 158c | 158cShortMainM20 | GAAAAACCTCGGAACGAGGGTAGCTTTTCTACCTGCACTAT AAGCACTTTA | 270 |
| 161c | 161cLong | GCAGCGAAAGACAGCAGTCTATCAGGGCGATGTAGGGTTG | 271 |
| 162s | 162sBothLongM20 | GTGCAGGTAGTTTTTGACCTGAAAGCGTAAGGGATCGTCA CCCTCATTTTCTACCTGCACTATAAGCACTTTA | 272 |

Staple DNA with a staple number labeled "Telo" contains a telomere element sequence (including two G triplet repeats). The structure formed by the telomere element sequence, its position, etc. are the same as those in Reference Example 2. The telomere element sequence in the partial structure 1 and the telomere element sequence in the partial structure 2 can be linked in the presence of miR-20 and a monovalent cation to form a G-quartet structure (nucleic acid enzyme: heme stacks can exhibit peroxidase (HRP) activity).

Staple DNA with a staple number labeled "M20" contains the complementary sequence of miR-20 or a sequence complementary to part of the sequence. These sequences form a protruding structure consisting of single-stranded DNA without complementary binding to M13mp18. Among the three structures based on the conformation of the Holliday junction (Fig. 9: provided that Cross is an intermediate structure that mediates structural changes between Antiparallel and Parallel), when taking Antiparallel, the protruding structure in the partial structure 1 and the protruding structure in the partial structure 2 are arranged so that these protruding structures face each other (i.e., staple DNA containing the complementary sequence of miR-20 or a sequence complementary to part of the sequence is arranged). The complementary sequence of miR-20 in the partial structure 2 and the sequence complementary to part of the sequence in the partial structure 1 can be bonded based on complementary base pairing in the absence of miR-20.

That is, as shown in Fig. 13, the nucleic acid structure of this Example is fixed at the antiparallel type based on complementary binding in the absence of miR-20, whereas in the presence of miR-20, the complementary bond dissociates to induce a structural change to the cross type, and the presence of a monovalent cation induces a structure change to the parallel type based on the formation of a G-quartet structure, resulting in the exhibition of peroxidase activity.

A KCl solution (1 M KCl was dissolved in 1×TAE/Mg²⁺ solution) was added to a solution containing the prepared nucleic acid structure, followed by still standing for 5 minutes. Then, a test substance, miR20 (dissolved in RNase-free Water (catalog number: 9012, TaKaRa) at 200 µM), was added, followed by still standing for 1 hour. AFM observation was then performed, and the presence of miR20 was visualized as a structural difference of the nucleic acid structure. The solution containing the nucleic acid structure contained 3.2 nM of the nucleic acid structure, 1 µM of miR20, and 200 mM of KCl. Further, each structure of the nucleic acid structure was counted from the acquired images, and its proportion was examined.

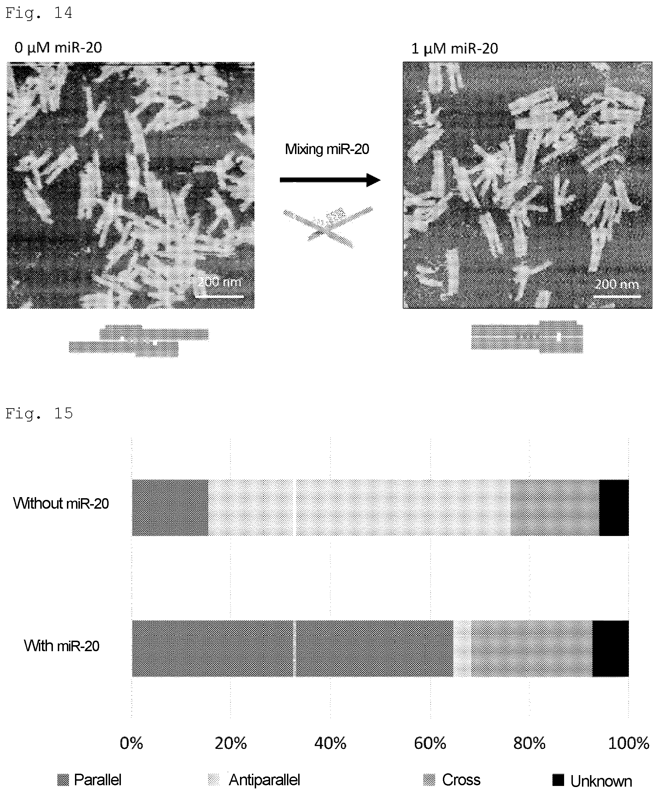

Fig. 14 shows AFM observation images. Further, Fig. 15 shows the proportion of each structure. The antiparallel type was the majority before the addition of miR-20, indicating that the addition of miR-20 induced a structural change from the antiparallel type to the parallel type.

### Example 2: Detection 2 of Enzymatic Activity

A KCl solution (1 M KCl was dissolved in 1×TAE/Mg²⁺ solution) was added to a solution containing the nucleic acid structure (Example 1), followed by still standing for 5 minutes. Then, a test substance, miR20 (dissolved in RNase-free Water (catalog number: 9012, TaKaRa) at 200 µM), was added, followed by still standing for 1 hour. Further, hemin (dissolved in DMSO at 100 µM) was added, followed by still standing for 1 hour. The solution containing the nucleic acid structure contained 3.2 nM of the nucleic acid structure, 10 µM of miR20, 4.0 µM of hemin, and 200 mM of KCl. Then, an equivalent amount of 1-Step^{™} Ultra TMB-ELISA (catalog number: 34028, Thermo Scientific) was added, and the presence of miR20 was visualized as a color.

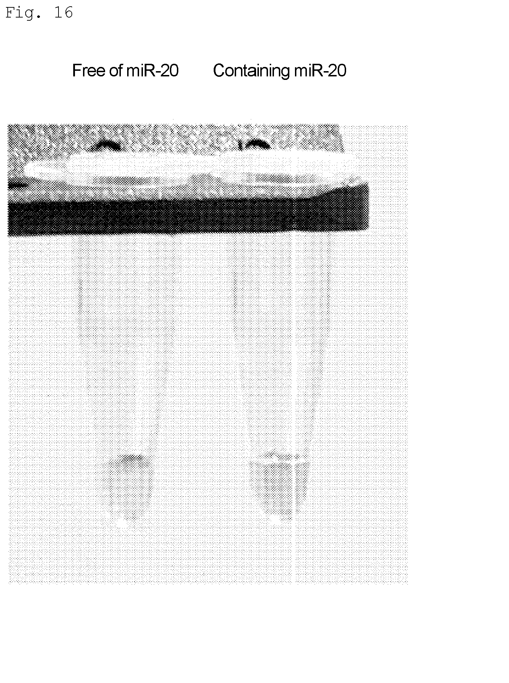

The results are shown in Fig. 16. A strong blue color was developed when miR-20 was contained. This revealed that miR-20 can be detected as a color change using the nucleic acid structure.

In conventional DNA nano-structures, split G-quartet sequences are highly stable, and it is difficult to maintain the split state; however, in the present invention, the pre-fixation on the opposite side (antiparallel type) successfully prevented the leakage of enzymatic activity. By using the technique of the present invention, the enzymatic activity can be controlled, for example, by varying the number of DNA strands attached to biomolecules. Unlike immunochromatography, the technique of the present invention is superior in that it does not require the fixation of antibodies or aptamers to underlying membranes or the like. In addition, the technique of the present invention, which can be composed only of nucleic acids, allows mass synthesis in a short time, cheaply and easily, with less lot differences compared to antibodies.

### Example 3: Preparation 4 of Nucleic Acid Structure

A nucleic acid structure was prepared in the same manner as in Reference Example 1, except that some staple DNAs were changed to other staple DNAs. Tables 10 and 11 show the corresponding staple numbers of Reference Example 1, and the staple numbers, base sequences, and SEQ ID Nos of the staple DNAs used. The configuration of the obtained nucleic acid structure was the same as that of the nucleic acid structure of Reference Example 1, except that the partial structure 1 and the partial structure 2 each contained a protruding structure consisting of single-stranded DNA of a telomere element sequence, the partial nucleic acid structure 1 contained a protruding structure containing a nucleic acid aptamer sequence (complementary sequence of R509 (SEQ ID NO: 297)), and the partial nucleic acid structure 2 contained a sequence complementary to part of the nucleic acid aptamer sequence. R509 is the complementary sequence of NIID_WH-1_F509 (National Institute of Infectious Diseases, Manual for the Detection of Pathogen 2019-nCoV Ver. 2.9.1).

**Table 10**

| Corresponding staple No. of Ref. Ex. 1 | staple No. | Sequence | SEQ ID No |
|---|---|---|---|
| 65 | 65long | TCTTAAACAGCTTGATGTGCCGTCGAGAGGGTGAGCCGCC | 275 |
| 66 | 66shortTelo | GTATAGCCGCTTTCGAGGTGATTTTTTTAGGGTTAGGGT | 276 |
| 69 | 69long | ACAATGACAACAACCATAGCGGGGTTTTGCTCCAGGTCAG | 277 |
| 70 | 70shortTelo | GCGGATAAACCGATAGTTGCGCCGTTTTTAGGGTTAGGGT | 278 |
| 73 | 73LongCom pCF509 | CAGTTCGAGTTTTGATATATTCGGTCGCTAGGCTGAGACTCCT CACAAATAAA | 279 |
| 74 | 74shortTelo | ATTAGGATTCGCCCACGCATAACCTTTTTAGGGTTAGGGT | 280 |
| 78s | 78s-telo | GTATTAAGGAGGCTTGCAGGGAGTTTTTTAGGGTTAGGGT | 281 |
| 166s | 166s-telo | GTCGAGGTAGAGATAGAACCCTTCTTTTTAGGGTTAGGGT | 282 |
| 169 | 169LongCompCF509 | CAGTTCGAGTTTTGGAACATTTCTGGCCAACGCCGTAAAGCAC TAAAATCCTGTT | 283 |
| 170 | 170shortTelo | CTAAAGGGCGACCAGTAATAAAAGTTTTTAGGGTTAGGGT | 284 |
| 173 | 173long | AGATTCACCAGTCACAAGCCCCCGATTTAGAGCGGTCCAC | 285 |
| 174 | 174shortTelo | GGAAAGCCGATTATTTACATTGGCTTTTTAGGGTTAGGGT | 286 |
| 177 | 177long | TCAATCGTCTGAAATGGGCGAACGTGGCGAGATCACCGCC | 287 |
| 178 | 178shortTelo | GAAGAAAGACCTACATTTTGACGCTTTTTAGGGTTAGGGT | 288 |

**Table 11**

| Corresponding staple No. of Ref. Ex. 1 | staple No. | Sequence | SEQ ID No |
|---|---|---|---|
| 81c | 81cLong | CAATATTTTTGAATGGTATAAACAGTTAATGCGTCATACA | 289 |
| 82s | 82sBothLongCF509 | CAGTTCGAGTTTTTAAAGGCCGCTTTTGCGAATACGTGGCACA GATTTTCTCGAACTGCACCTCATGG | 290 |
| 85c | 85cLong | CGCGAACTGATAGCCCAACGGGGTCAGTGCCTACTGGTAA | 291 |
| 86c | 86cShortCF509 | AGTGCCCGCTATTAGTCTTTAATGTTTTCTCGAACTGCACCTCA TGG | 292 |
| 157c | 157cLong | AAACGGCTACAGAGGCTACGTGGACTCCAACGTAGTCCACT | 293 |
| 158c | 158cShortCF509 | GAAAAACCTCGGAACGAGGGTAGCTTTTCTCGAACTGCACCTC ATGG | 294 |
| 161c | 161cLong | GCAGCGAAAGACAGCAGTCTATCAGGGCGATGTAGGGTTG | 295 |
| 162s | 162sBothLongCF509 | CAGTTCGAGTTTTTGACCTGAAAGCGTAAGGGATCGTCACCCT CATTTTCTCGAACTGCACCTCATGG | 296 |

Staple DNA with a staple number labeled "Telo" contains a telomere element sequence (including two G triplet repeats). The structure formed by the telomere element sequence, its position, etc. are the same as those in Reference Example 2. The telomere element sequence in the partial structure 1 and the telomere element sequence in the partial structure 2 can be linked in the presence of R509 and a monovalent cation to form a G-quartet structure (nucleic acid enzyme: heme stacks can exhibit peroxidase (HRP) activity).

Staple DNA with a staple number labeled "CF509" contains the complementary sequence of R509 or a sequence complementary to part of the sequence. These sequences form a protruding structure consisting of single-stranded DNA without complementary binding to M13mp18. Among the three structures based on the conformation of the Holliday junction (Fig. 9: provided that Cross is an intermediate structure that mediates structural changes between Antiparallel and Parallel), when taking Antiparallel, the protruding structure in the partial structure 1 and the protruding structure in the partial structure 2 are arranged so that these protruding structures face each other (i.e., staple DNA containing the complementary sequence of R509 or a sequence complementary to part of the sequence is arranged). The complementary sequence of R509 in the partial structure 2 and the sequence complementary to part of the sequence in the partial structure 1 can be bonded based on complementary base pairing in the absence of R509.

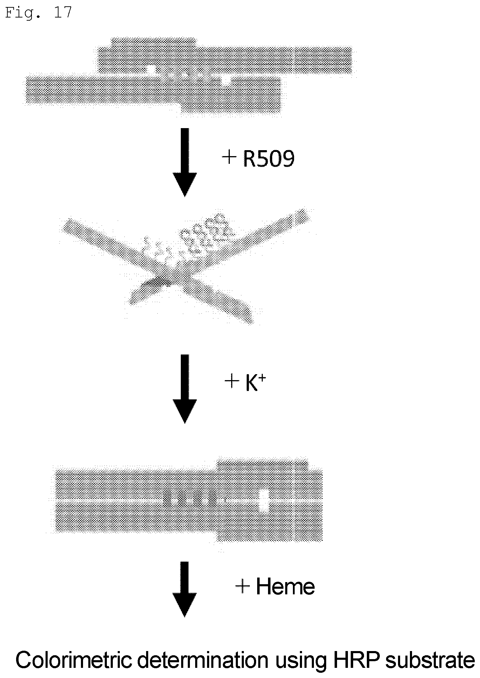

That is, as shown in Fig. 17, the nucleic acid structure of this Example is fixed at the antiparallel type based on complementary binding in the absence of R509, whereas in the presence of R509, the complementary bond dissociates to induce a structural change to the cross type, and the presence of a monovalent cation induces a structure change to the parallel type based on the formation of a G-quartet structure, resulting in the exhibition of peroxidase activity.

A KCl solution (1 M KCl was dissolved in 1×TAE/Mg²⁺ solution) was added to a solution containing the prepared nucleic acid structure, followed by still standing for 5 minutes. Then, a test substance, R509 (dissolved in RNase-free Water (catalog number: 9012, TaKaRa) at 200 µM), or R29142 (SEQ ID NO: 344), which was not a test substance, was added, followed by still standing for 1 hour. AFM observation was then performed, and the presence of R509 was visualized as a structural difference of the nucleic acid structure. The solution containing the nucleic acid structure contained 3.2 nM of the nucleic acid structure, 1 µM of R509, and 200 mM of KCl. Further, each structure of the nucleic acid structure was counted from the acquired images, and its proportion was examined. R29142 is the complementary sequence of NIID_2019-nCOV_N_F2 (National Institute of Infectious Diseases, Manual for the Detection of Pathogen 2019-nCoV Ver. 2.9.1).

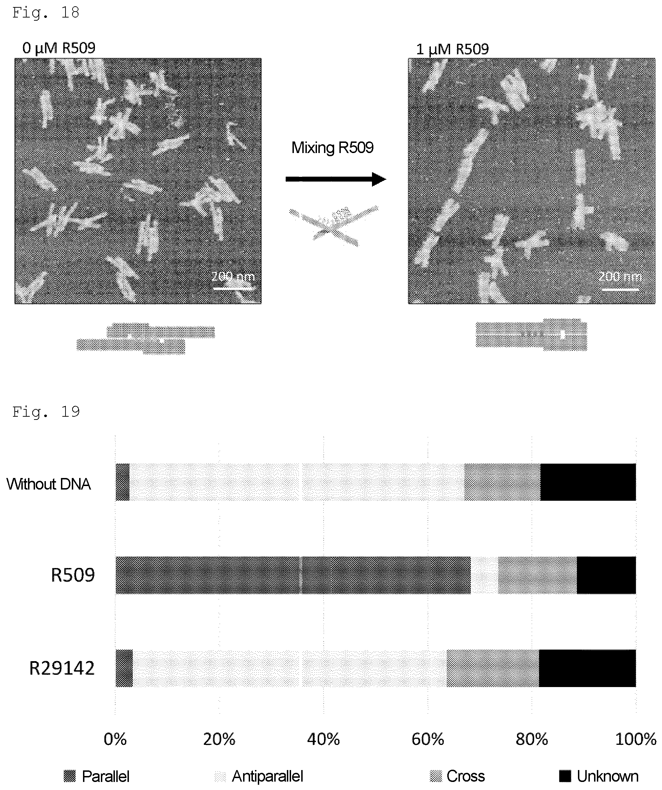

Fig. 18 shows AFM observation images. Further, Fig. 19 shows the proportion of each structure. The antiparallel type was the majority before the addition of R509, indicating that the addition of R509 induced a structural change from the antiparallel type to the parallel type. It was also indicated that the addition of R29142 did not induce a structural change from the antiparallel type to the parallel type.

### Example 4: Detection 3 of Enzymatic Activity

A KCl solution (1 M KCl was dissolved in 1×TAE/Mg²⁺ solution) was added to a solution containing the nucleic acid structure (Example 3), followed by still standing for 5 minutes. Then, a test substance, R509 (dissolved in RNase-free Water (catalog number: 9012, TaKaRa) at 200 µM), was added, followed by still standing for 1 hour. Further, hemin (dissolved in DMSO at 100 µM) was added, followed by still standing for 1 hour. The solution containing the nucleic acid structure contained 3.2 nM of the nucleic acid structure, 10 µM of R509, 4.0 µM of hemin, and 200 mM of KCl. Then, an equivalent amount of 1-Step^{™} Ultra TMB-ELISA (catalog number: 34028, Thermo Scientific) was added, and the presence of R509 was visualized as a color.

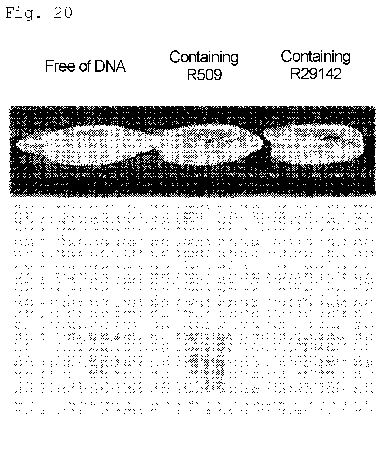

The results are shown in Fig. 20. A strong blue color was developed when R509 was contained. This revealed that R509 can be detected as a color change using the nucleic acid structure.

In conventional DNA nano-structures, split G-quartet sequences are highly stable, and it is difficult to maintain the split state; however, in the present invention, the pre-fixation on the opposite side (antiparallel type) successfully prevented the leakage of enzymatic activity. By using the technique of the present invention, the enzymatic activity can be controlled, for example, by varying the number of DNA strands attached to biomolecules. Unlike immunochromatography, the technique of the present invention is superior in that it does not require the fixation of antibodies or aptamers to underlying membranes or the like. In addition, the technique of the present invention, which can be composed only of nucleic acids, allows mass synthesis in a short time, cheaply and easily, with less lot differences compared to antibodies.

### Example 5: Preparation 5 of Nucleic Acid Structure

A nucleic acid structure was prepared in the same manner as in Reference Example 1, except that some staple DNAs were changed to other staple DNAs. Tables 12 and 13 show the corresponding staple numbers of Reference Example 1, and the staple numbers, base sequences, and SEQ ID Nos of the staple DNAs used. The configuration of the obtained nucleic acid structure was the same as that of the nucleic acid structure of Reference Example 1, except that the partial structure 1 and the partial structure 2 each contained a protruding structure consisting of single-stranded DNA of a telomere element sequence, the partial nucleic acid structure 1 contained a protruding structure containing a nucleic acid aptamer sequence (complementary sequence of N1 (SEQ ID NO: 320)), and the partial nucleic acid structure 2 contained a sequence complementary to part of the nucleic acid aptamer sequence. The complementary sequence of N1 is a sequence complementary to the nucleic acid sequence of N_Sarbeco_P1 (National Institute of Infectious Diseases, Manual for the Detection of Pathogen 2019-nCoV Ver. 2.9.1).

**Table 12**

| Corresponding staple No. of Ref. Ex. 1 | staple No. | Sequence | SEQ ID No |
|---|---|---|---|
| 65 | 65long | TCTTAAACAGCTTGATGTGCCGTCGAGAGGGTGAGCCGCC | 298 |
| 66 | 66shortTelo | GTATAGCCGCTTTCGAGGTGATTTTTTTAGGGTTAGGGT | 299 |
| 69 | 69long | ACAATGACAACAACCATAGCGGGGTTTTGCTCCAGGTCAG | 300 |
| 70 | 70shortTelo | GCGGATAAACCGATAGTTGCGCCGTTTTTAGGGTTAGGGT | 301 |
| 73 | 73LongCompN1 | AACATTGCCATTTTGATATATTCGGTCGCTAGGCTGAGACTCCT CACAAATAAA | 302 |
| 74 | 74shortTelo | ATTAGGATTCGCCCACGCATAACCTTTTTAGGGTTAGGGT | 303 |
| 78s | 78s-telo | GTATTAAGGAGGCTTGCAGGGAGTTTTTTAGGGTTAGGGT | 304 |
| 166s | 166s-telo | GTCGAGGTAGAGATAGAACCCTTCTTTTTAGGGTTAGGGT | 305 |
| 169 | 169LongCompN1 | AACATTGCCATTTTGGACATTCTGGCCAACGCCGTAAAGCACTA AAATCCTGTT | 306 |
| 170 | 170shortTelo | CTAAAGGGCGACCAGTAATAAAAGTTTTTAGGGTTAGGGT | 307 |
| 173 | 173long | AGATTCACCAGTCACAAGCCCCCGATTTAGAGCGGTCCAC | 308 |
| 174 | 174shortTelo | GGAAAGCCGATTATTTACATTGGCTTTTTAGGGTTAGGGT | 309 |
| 177 | 177long | TCAATCGTCTGAAATGGGCGAACGTGGCGAGATCACCGCC | 310 |
| 178 | 178shortTelo | GAAGAAAGACCTACATTTTGACGCTTTTTAGGGTTAGGGT | 311 |

**Table 13**

| Corresponding staple No. of Ref. Ex. 1 | staple No. | Sequence | SEQ ID No |
|---|---|---|---|
| 81c | 81cLong | CAATATTTTTGAATGGTATAAACAGTTAATGCGTCATACA | 312 |
| 82s | 82sBothLongN1 | AACATTGCCATTTTTAAAGGCCGCTTTTGCGAATACGTGGCACA GATTTTTGGCAATGTTGTTCCTTGAGGAAGT | 313 |
| 85c | 85cLong | CGCGAACTGATAGCCCAACGGGGTCAGTGCCTACTGGTAA | 314 |
| 86c | 86cShortN1 | AGTGCCCGCTATTAGTCTTTAATGTTTTTGGCAATGTTGTTCCT TGAGGAAGT | 315 |
| 157c | 157cLong | AAACGGCTACAGAGGCTACGTGGACTCCAACGTAGTCCACT | 316 |
| 158c | 158cShortN1 | GAAAAACCTCGGAACGAGGGTAGCTTTTTGGCAATGTTGTTCC TTGAGGAAGT | 317 |
| 161c | 161cLong | GCAGCGAAAGACAGCAGTCTATCAGGGCGATGTAGGGTTG | 318 |
| 162s | 162sBothLongN1 | AACATTGCCATTTTTGACCTGAAAGCGTAAGGGATCGTCACCC TCATTTTTGGCAATGTTGTTCCTTGAGGAAGT | 319 |

Staple DNA with a staple number labeled "Telo" contains a telomere element sequence (including two G triplet repeats). The structure formed by the telomere element sequence, its position, etc. are the same as those in Reference Example 2. The telomere element sequence in the partial structure 1 and the telomere element sequence in the partial structure 2 can be linked in the presence of N1 and a monovalent cation to form a G-quartet structure (nucleic acid enzyme: heme stacks can exhibit peroxidase (HRP) activity).

Staple DNA with a staple number labeled "N1" contains the complementary sequence of N1 or a sequence complementary to part of the sequence. These sequences form a protruding structure consisting of single-stranded DNA without complementary binding to M13mp18. Among the three structures based on the conformation of the Holliday junction (Fig. 9: provided that Cross is an intermediate structure that mediates structural changes between Antiparallel and Parallel), when taking Antiparallel, the protruding structure in the partial structure 1 and the protruding structure in the partial structure 2 are arranged so that these protruding structures face each other (i.e., staple DNA containing the complementary sequence of N1 or a sequence complementary to part of the sequence is arranged). The complementary sequence of N1 in the partial structure 2 and the sequence complementary to part of the sequence in the partial structure 1 can be bonded based on complementary base pairing in the absence of N1.

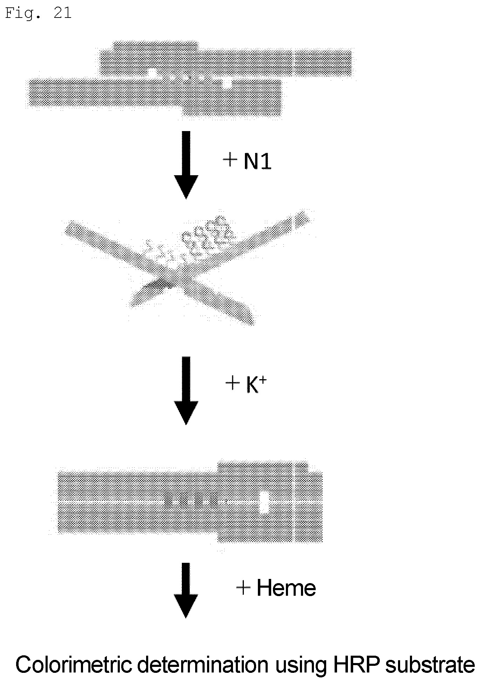

That is, as shown in Fig. 21, the nucleic acid structure of this Example is fixed at the antiparallel type based on complementary binding in the absence of N1, whereas in the presence of N1, the complementary bond dissociates to induce a structural change to the cross type, and the presence of a monovalent cation induces a structure change to the parallel type based on the formation of a G-quartet structure, resulting in the exhibition of peroxidase activity.

A KCl solution (1 M KCl was dissolved in 1×TAE/Mg²⁺ solution) was added to a solution containing the prepared nucleic acid structure, followed by still standing for 5 minutes. Then, a test substance, N1 (dissolved in RNase-free Water (catalog number: 9012, TaKaRa) at 200 µM), was added, followed by still standing for 1 hour. AFM observation was then performed, and the presence of N1 was visualized as a structural difference of the nucleic acid structure. The solution containing the nucleic acid structure contained 3.2 nM of the nucleic acid structure, 1.0 µM of N1, and 200 mM of KCl. Further, each structure of the nucleic acid structure was counted from the acquired images, and its proportion was examined.

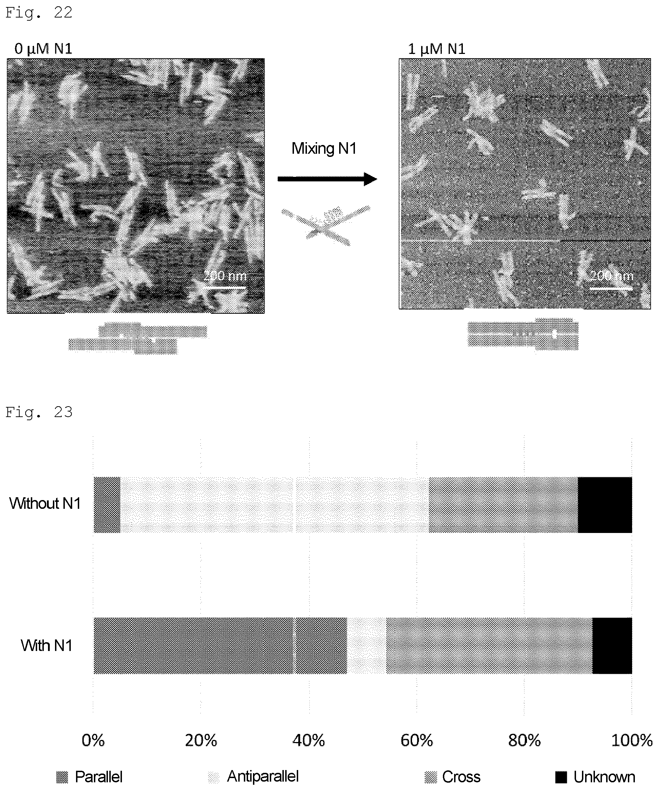

Fig. 22 shows AFM observation images. Further, Fig. 23 shows the proportion of each structure. The antiparallel type was the majority before the addition of N1, indicating that the addition of N1 induced a structural change from the antiparallel type to the parallel type.

### Example 6: Detection 4 of Enzymatic Activity

A KCl solution (1 M KCl was dissolved in 1×TAE/Mg²⁺ solution) was added to a solution containing the nucleic acid structure (Example 5), followed by still standing for 5 minutes. Then, a test substance, N1 (dissolved in RNase-free Water (catalog number: 9012, TaKaRa) at 200 µM), was added, followed by still standing for 15 minutes. Further, hemin (dissolved in DMSO at 100 µM) was added, followed by still standing for 30 minutes. The solution containing the nucleic acid structure contained 7.5 nM of the nucleic acid structure, 2.0 µM of N1, 5.0 µM of hemin, 0.0004% (w/v) of Triton X-100, and 200 mM of KCl. Then, an equivalent amount of 1-Step^{™} Ultra TMB-ELISA (catalog number: 34028, Thermo Scientific, pH: 4.9) mixed with 1×TAE/Mg²⁺ and 200 mM of KCl was added (pH of the solution after mixing: 5.5), and 10 minutes later, the presence of N1 was visualized as a color.

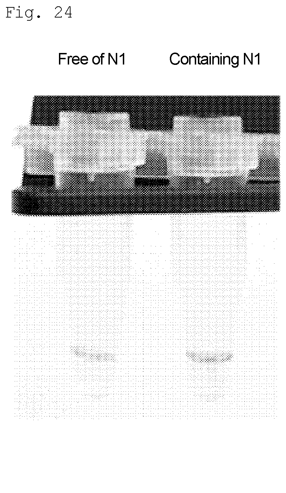

The results are shown in Fig. 24. A strong blue color was developed when N1 was contained. This revealed that N1 can be detected as a color change using the nucleic acid structure.

In conventional DNA nano-structures, split G-quartet sequences are highly stable, and it is difficult to maintain the split state; however, in the present invention, the pre-fixation on the opposite side (antiparallel type) successfully prevented the leakage of enzymatic activity. By using the technique of the present invention, the enzymatic activity can be controlled, for example, by varying the number of DNA strands attached to biomolecules. Unlike immunochromatography, the technique of the present invention is superior in that it does not require the fixation of antibodies or aptamers to underlying membranes or the like. In addition, the technique of the present invention, which can be composed only of nucleic acids, allows mass synthesis in a short time, cheaply and easily, with less lot differences compared to antibodies.

### Example 7: Preparation 6 of Nucleic Acid Structure

A nucleic acid structure was prepared in the same manner as in Reference Example 1, except that some staple DNAs were changed to other staple DNAs. Tables 14 and 15 show the corresponding staple numbers of Reference Example 1, and the staple numbers, base sequences, and SEQ ID Nos of the staple DNAs used. The configuration of the obtained nucleic acid structure was the same as that of the nucleic acid structure of Reference Example 1, except that the partial structure 1 and the partial structure 2 each contained a protruding structure consisting of single-stranded DNA of a telomere element sequence, the partial nucleic acid structure 1 contained a protruding structure containing a nucleic acid aptamer sequence (HD1 (thrombin aptamer, Bock, L. et al., (1992) Nature 355, 564-566, Munzar J. D. et al., (2018) Nat Commun. doi: 10.1038/s41467-017-02556-3.): SEQ ID NO: 343), and the partial nucleic acid structure 2 contained a sequence complementary to part of the nucleic acid aptamer sequence.

**Table 14**

| Corresponding staple No. of Ref. Ex. 1 | staple No. | Sequence | SEQ ID No |
|---|---|---|---|
| 65 | 65long | TCTTAAACAGCTTGATGTGCCGTCGAGAGGGTGAGCCGCC | 321 |
| 66 | 66shortTelo | GTATAGCCGCTTTCGAGGTGAATTTTTTTAGGGTTAGGGT | 322 |
| 69 | 69long | ACAATGACAACAACCATAGCGGGGTTTTGCTCCAGGTCAG | 323 |
| 70 | 70shortTelo | GCGGATAAACCGATAGTTGCGCCGTTTTTAGGGTTAGGGT | 324 |
| 73 | 73LongCompHD1T1 | ACACCAACCTTTTTGATATATTCGGTCGCTAGGCTGAGACTCCT CACAAATAAA | 325 |
| 74 | 74shortTelo | ATTAGGATTCGCCCACGCATAACCTTTTTAGGGTTAGGGT | 326 |
| 78s | 78s-telo | GTATTAAGGAGGCTTGCAGGGAGTTTTTTAGGGTTAGGGT | 327 |
| 166s | 166s-telo | GTCGAGGTAGAGATAGAACCCTTCTTTTTAGGGTTAGGGT | 328 |
| 169 | 159LongCompHD1T1 | ACACCAACCTTTTTGGACATTCTGGCCAACGCCGTAAAGCACTA AAATCCTGTT | 329 |
| 170 | 170shortTelo | CTAAAGGGCGACCAGTAATAAAAGTTTTTAGGGTTAGGGT | 330 |
| 173 | 173long | AGATTCACCAGTCACAAGCCCCCGATTTAGAGCGGTCCAC | 331 |
| 174 | 174shortTelo | GGAAAGCCGATTATTTACATTGGCTTTTTAGGGTTAGGGT | 332 |
| 177 | 177long | TCAATCGTCTGAAATGGGCGAACGTGGCGAGATCACCGCC | 333 |
| 178 | 178shortTelo | GAAGAAAGACCTACATTTTGACGCTTTTTAGGGTTAGGGT | 334 |

**Table 15**

| Corresponding staple No. of Ref. Ex. 1 | staple No. | Sequence | SEQ ID No |
|---|---|---|---|
| 81c | 81cLong | CAATATTTTTGAATGGTATAAACAGTTAATGCGTCATACA | 335 |
| 82s | 82sBothLongHD1T1 | ACACCAACCTTTTTTAAAGGCCGCTTTTGCGAATACGTGGCACA GATTTGTGGTTGGTGTGGTTGGTCATA | 336 |
| 85c | 85cLong | CGCGAACTGATAGCCCAACGGGGTCAGTGCCTACTGGTAA | 337 |
| 86c | 86cShortHD1T1 | AGTGCCCGCTATTAGTCTTTAATGTTTTAGTCCGTGGTAGGGC AGGTTGGGGTGACT | 338 |
| 157c | 157cLong | AAACGGCTACAGAGGCTACGTGGACTCCAACGTAGTCCACT | 339 |
| 158c | 158cShortHD1T1 | GAAAAACCTCGGAACGAGGGTAGCTTTTAGTCCGTGGTAGGGC AGGTTGGGGTGACT | 340 |
| 161c | 161cLong | GCAGCGAAAGACAGCAGTCTATCAGGGCGATGTAGGGTTG | 341 |
| 162s | 162sBothLongHD1T1 | ACACCAACCTTTTTTGACCTGAAAGCGTAAGGGATCGTCACCC TCATTTGTGGTTGGTGTGGTTGGTCATA | 342 |

Staple DNA with a staple number labeled "Telo" contains a telomere element sequence (including two G triplet repeats). The structure formed by the telomere element sequence, its position, etc. are the same as those in Reference Example 2. The telomere element sequence in the partial structure 1 and the telomere element sequence in the partial structure 2 can be linked in the presence of thrombin and a monovalent cation to form a G-quartet structure (nucleic acid enzyme: heme stacks can exhibit peroxidase (HRP) activity).

Staple DNA with a staple number labeled "HD1T1" contains the complementary sequence of HD1 or a sequence complementary to part of the sequence. These sequences form a protruding structure consisting of single-stranded DNA without complementary binding to M13mp18. Among the three structures based on the conformation of the Holliday junction (Fig. 9: provided that Cross is an intermediate structure that mediates structural changes between Antiparallel and Parallel), when taking Antiparallel, the protruding structure in the partial structure 1 and the protruding structure in the partial structure 2 are arranged so that these protruding structures face each other (i.e., staple DNA containing the complementary sequence of HD1T1 or a sequence complementary to part of the sequence is arranged). The complementary sequence of HD1T1 in the partial structure 2 and the sequence complementary to part of the sequence in the partial structure 1 can be bonded based on complementary base pairing in the absence of thrombin.

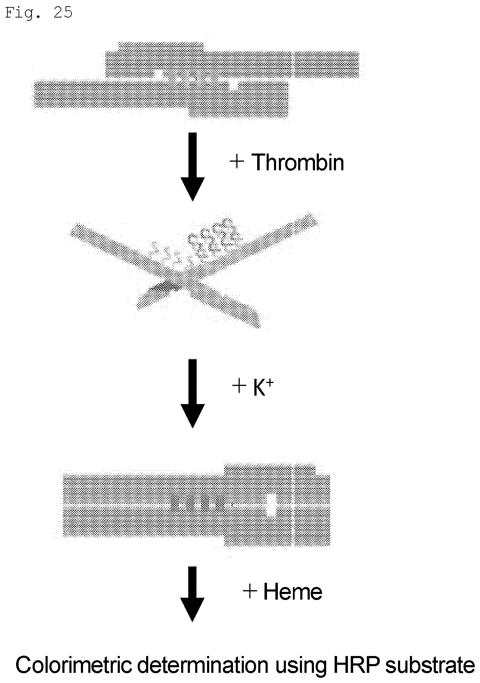

That is, as shown in Fig. 25, the nucleic acid structure of this Example is fixed at the antiparallel type based on complementary binding in the absence of thrombin, whereas in the presence of thrombin, the complementary bond dissociates to induce a structural change to the cross type, and the presence of a monovalent cation induces a structure change to the parallel type based on the formation of a G-quartet structure, resulting in the exhibition of peroxidase activity.

A KCl solution (1 M KCl was dissolved in 1×TAE/Mg²⁺ solution) was added to a solution containing the prepared nucleic acid structure, followed by still standing for 5 minutes. Then, a test substance, thrombin, was added, followed by still standing for 1 hour. AFM observation was then performed, and the presence of thrombin was visualized as a structural difference of the nucleic acid structure. The solution containing the nucleic acid structure contained 3.2 nM of the nucleic acid structure, 2.0 µM of thrombin, and 200 mM of KCl. Further, each structure of the nucleic acid structure was counted from the acquired images, and its proportion was examined.

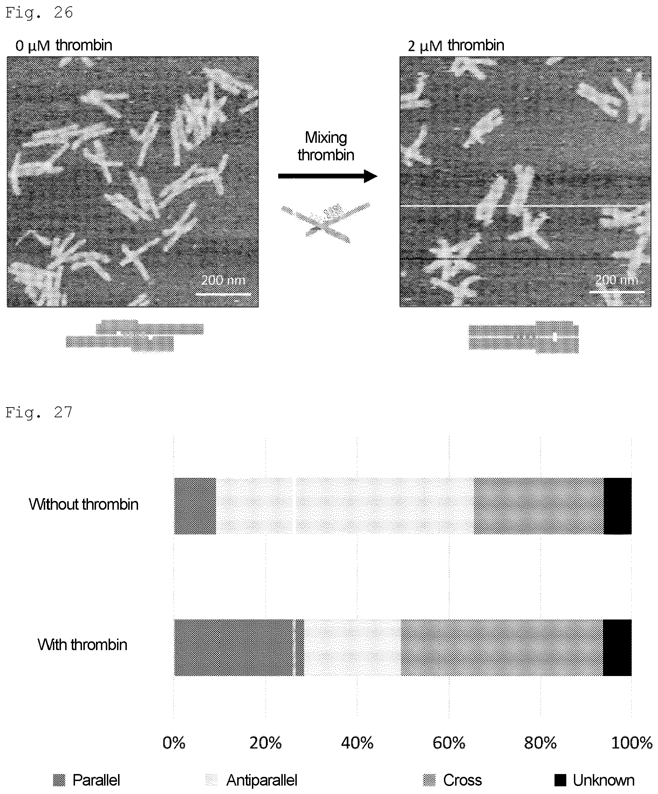

Fig. 26 shows AFM observation images. Further, Fig. 27 shows the proportion of each structure. The antiparallel type was the majority before the addition of thrombin, indicating that the addition of thrombin induced a structural change from the antiparallel type to the parallel type.

### Example 8: Detection 5 of Enzymatic Activity

A KCl solution (1 M KCl was dissolved in 1×TAE/Mg²⁺ solution) was added to a solution containing the nucleic acid structure (Example 7), followed by still standing for 5 minutes. Then, a test substance, thrombin, was added, followed by still standing for 15 minutes. Further, hemin (dissolved in DMSO at 100 µM) was added, followed by still standing for 30 minutes. The solution containing the nucleic acid structure contained 7.5 nM of the nucleic acid structure, 2.0 µM of thrombin, 5.0 µM of hemin, 0.0004% (w/v) of Triton X-100, and 200 mM of KCl. Then, an equivalent amount of 1-Step^{™} Ultra TMB-ELISA (catalog number: 34028, Thermo Scientific) was added, and the presence of thrombin was visualized as a color.

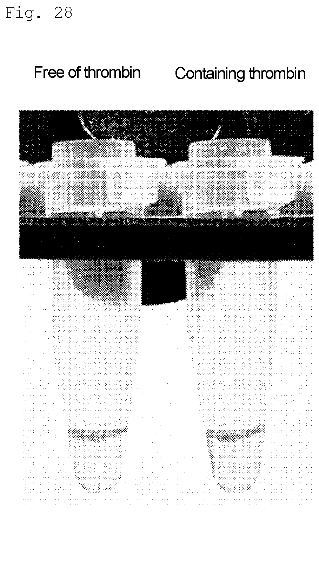

The results are shown in Fig. 28. A strong blue color was developed when thrombin was contained. This revealed that thrombin can be detected as a color change using the nucleic acid structure.

In conventional DNA nano-structures, split G-quartet sequences are highly stable, and it is difficult to maintain the split state; however, in the present invention, the pre-fixation on the opposite side (antiparallel type) successfully prevented the leakage of enzymatic activity. By using the technique of the present invention, the enzymatic activity can be controlled, for example, by varying the number of DNA strands attached to biomolecules. Unlike immunochromatography, the technique of the present invention is superior in that it does not require the fixation of antibodies or aptamers to underlying membranes or the like. In addition, the technique of the present invention, which can be composed only of nucleic acids, allows mass synthesis in a short time, cheaply and easily, with less lot differences compared to antibodies.

### Example 9: Preparation 7 of Nucleic Acid Structure

NanoLuc (Promega) is a luciferase with furimazine as a substrate, and its luminescence intensity is very strong, about 100 times the luminescence intensity of firefly luciferase. LgBiT (Promega) and SmBiT (Promega) are subunits of NanoLuc, and they are reconstituted as a luciferase by interacting with each other. LgBiT is a large subunit of approximately 18 kDa, and SmBiT is a small subunit of 11 amino acid residues. LgBiT and SmBiT have a dissociation constant of 190 µM and extremely low affinity, and they do not express luciferase activity when simply mixed (Andrew, D., (2016) ACS Chem. Biol. 11, 400-408). In this Example, only when the nucleic acid structure is structurally changed to the parallel type, SmBiT and LgBiT are brought close enough to interact, so that SmBiT and LgBiT interact only in the presence of a test substance, resulting in the exhibition of luciferase activity. Specifically, a nucleic acid structure was prepared in the following manner.

SmBiT and 95w (SEQ ID NO: 95) were bonded together. SmBiT (VTGYRLFEEIL-orn(N3)) modified with an azide group on the C-terminal side was chemically synthesized (Sigma-Aldrich). On the other hand, 95w modified with an amine on the 5-terminal side was chemically synthesized (IDT), and 1/30 equivalent (molar ratio) thereof was mixed with DBCO-NHS Ester (Click Chemistry Tools). Azide group-modified SmBiT and DBCO-modified 95w were mixed at a molar ratio of 1:1, and the mixture was allowed to stand for 16 hours and then purified by HPLC, thereby obtaining SmBiT-95w.

LgBiT and 138w (SEQ ID NO: 138) were bonded together. Lgbit was inserted into pH6HTN His6HaloTag T7 Vector (Promega) using the In-Fusion HD Cloning Kit (Clontech), and transformed into DH5α (SMOBIO Technology, Inc.) to obtain an LgBiT expression vector. The vector was transformed into BL21 (SMOBIO Technology, Inc.) and cultured in LB medium, and protein expression was induced with 0.4 mM of IPTG (Nacalai). The resultant was purified using the His-Spin Protein Miniprep Kit (Zymo Research), thereby obtaining HaloTag-fused LgBiT. On the other hand, 138w modified with an amine on the 5-terminal side was chemically synthesized (IDT), and 1/30 equivalent (molar ratio) thereof was mixed with HaloTag (registered trademark) Succinimidyl Ester (O4) Ligand (Promega). The mixture was allowed to stand for 16 hours, and then purified by HPLC, thereby obtaining HaloTag ligand-binding 138w staple. HaloTag-fused LgBiT and HaloTag ligand-binding 138w staple were mixed at a molar ratio of 1:1, and the mixture was allowed to stand for 16 hours. Then, unreacted 138w staple was removed using a centrifugal ultrafiltration filter (Amicon Ultra 0.5 mL-50 K, Millipore), thereby obtaining LgBiT-138w.

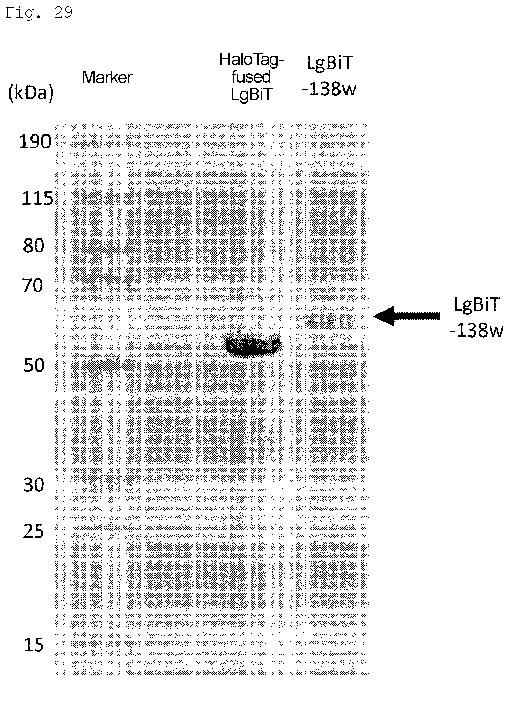

The obtained Lgbit-138w was confirmed by SDS polyacrylamide electrophoresis. Fig. 29 shows an electrophoretic image. From left, a marker (PageRuler Plus Prestain Protein Ladder, Thermo Scientific), HaloTag-fused LgBiT, and LgBiT-138w are shown. HaloTag-fused LgBiT migrated at a position similar to its expected molecular weight (approximately 54 kDa). In addition, the increase in molecular weight was confirmed based on the binding procedure of 138w shown above, suggesting that 138w bound to LgBiT.

A nucleic acid structure was prepared in the same manner as in Reference Example 1, except that some staple DNAs were changed to other staple DNAs. Tables 16 and 17 show the corresponding staple numbers of Reference Example 1, and the staple numbers, base sequences, and SEQ ID Nos of the staple DNAs used. LgBiT-138w was added at a molar ratio of 1:1 after the nucleic acid structure was prepaired with other staple DNAs except for this. The configuration of the obtained nucleic acid structure was the same as that of the nucleic acid structure of Reference Example 1, except that the partial structure 1 and the partial structure 2 each contained a protruding structure consisting of single-stranded DNA of a telomere element sequence, the partial nucleic acid structure 1 contained a protruding structure containing a nucleic acid aptamer sequence (complementary sequence of miR-20 (Mature sequence hsa-miR-20a-5p: SEQ ID NO: 274)), and the partial nucleic acid structure 2 contained a sequence complementary to part of the nucleic acid aptamer sequence, and contained SmbiT and LgBiT.

**Table 16**

| Corresponding staple No. of Ref. Ex. 1 | staple No. | Sequence | SEQ ID No |
|---|---|---|---|
| 65 | 65long | TCTTAAACAGCTTGATGTGCCGTCGAGAGGGTGAGCCGCC | 345 |
| 66 | 66shortTelo | GTATAGCCGCTTTCGAGGTGAATTTTTTTAGGGTTAGGGT | 346 |
| 69 | 69long | ACAATGACAACAACCATAGCGGGGTTTTGCTCCAGGTCAG | 347 |
| 70 | 70shortTelo | GCGGATAAACCGATAGTTGCGCCGTTTTTAGGGTTAGGGT | 348 |
| 73 | 73LongCompM20 | GTGCAGGTAGTTTTGATATATTCGGTCGCTAGGCTGAGACTCC TCACAAATAAA | 349 |
| 74 | 74shortTelo | ATTAGGATTCGCCCACGCATAACCTTTTTAGGGTTAGGGT | 350 |
| 78s | 78s-telo | GTATTAAGGAGGCTTGCAGGGAGTTTTTTAGGGTTAGGGT | 351 |
| 166s | 166s-telo | GTCGAGGTAGAGATAGAACCCTTCTTTTTAGGGTTAGGGT | 352 |
| 169 | 169LongCompM20 | GTGCAGGTAGTTTTGGAACATTTCTGGCCAACGCCGTAAAGCA CTAAAATCCTGTT | 353 |
| 170 | 170shortTelo | CTAAAGGGCGACCAGTAATAAAAGTTTTTAGGGTTAGGGT | 354 |
| 173 | 173long | AGATTCACCAGTCACAAGCCCCCGATTTAGAGCGGTCCAC | 355 |
| 174 | 174shortTelo | GGAAAGCCGATTATTTACATTGGCTTTTTAGGGTTAGGGT | 356 |
| 177 | 177long | TCAATCGTCTGAAATGGGCGAACGTGGCGAGATCACCGCC | 357 |
| 178 | 178shortTelo | GAAGAAAGACCTACATTTTGACGCTTTTTAGGGTTAGGGT | 358 |

**Table 17**

| Corresponding staple No. of Ref. Ex. 1 | staple No. | Sequence | SEQ ID No |
|---|---|---|---|
| 81c | 81cLong | CAATATTTTTGAATGGTATAAACAGTTAATGCGTCATACA | 359 |
| 82s | 82sBothLongM20 | GTGCAGGTAGTTTTTAAAGGCCGCTTTTGCGAATACGTGGCAC AGATTTTCTACCTGCACTATAAGCACTTTA | 360 |
| 85c | 85cLong | CGCGAACTGATAGCCCAACGGGGTCAGTGCCTACTGGTAA | 361 |
| 86c | 86cShortMainM20 | AGTGCCCGCTATTAGTCTTTAATGTTTTCTACCTGCACTATAAG CACTTTA | 362 |
| 157c | 157cLong | AAACGGCTACAGAGGCTACGTGGACTCCAACGTAGTCCACT | 363 |
| 158c | 158cShortMainM20 | GAAAAACCTCGGAACGAGGGTAGCTTTTCTACCTGCACTATAA GCACTTTA | 364 |
| 161c | 161cLong | GCAGCGAAAGACAGCAGTCTATCAGGGCGATGTAGGGTTG | 365 |
| 162s | 162sBothLongM20 | GTGCAGGTAGTTTTTGACCTGAAAGCGTAAGGGATCGTCACCC TCATTTTCTACCTGCACTATAAGCACTTTA | 366 |
| 95w | SmBiT-95w | SmBiT-AAGGAATTGAGGAAGG | 367 |
| 138w | LgBiT-138w | LgBiT-ATCCGCGACCTGCTCC | 368 |

Staple DNA with a staple number labeled "Telo" contains a telomere element sequence (including two G triplet repeats). The structure formed by the telomere element sequence, its position, etc. are the same as those in Reference Example 2. The telomere element sequence in the partial structure 1 and the telomere element sequence in the partial structure 2 can be linked in the presence of miR-20 and a monovalent cation to form a G-quartet structure.

Staple DNA with a staple number labeled "M20" contains the complementary sequence of miR-20 or a sequence complementary to part of the sequence. These sequences form a protruding structure consisting of single-stranded DNA without complementary binding to M13mp18. Among the three structures based on the conformation of the Holliday junction (Fig. 9: provided that Cross is an intermediate structure that mediates structural changes between Antiparallel and Parallel), when taking Antiparallel, the protruding structure in the partial structure 1 and the protruding structure in the partial structure 2 are arranged so that these protruding structures face each other (i.e., staple DNA containing the complementary sequence of miR-20 or a sequence complementary to part of the sequence is arranged). The complementary sequence of miR-20 in the partial structure 2 and the sequence complementary to part of the sequence in the partial structure 1 can be bonded based on complementary base pairing in the absence of miR-20.

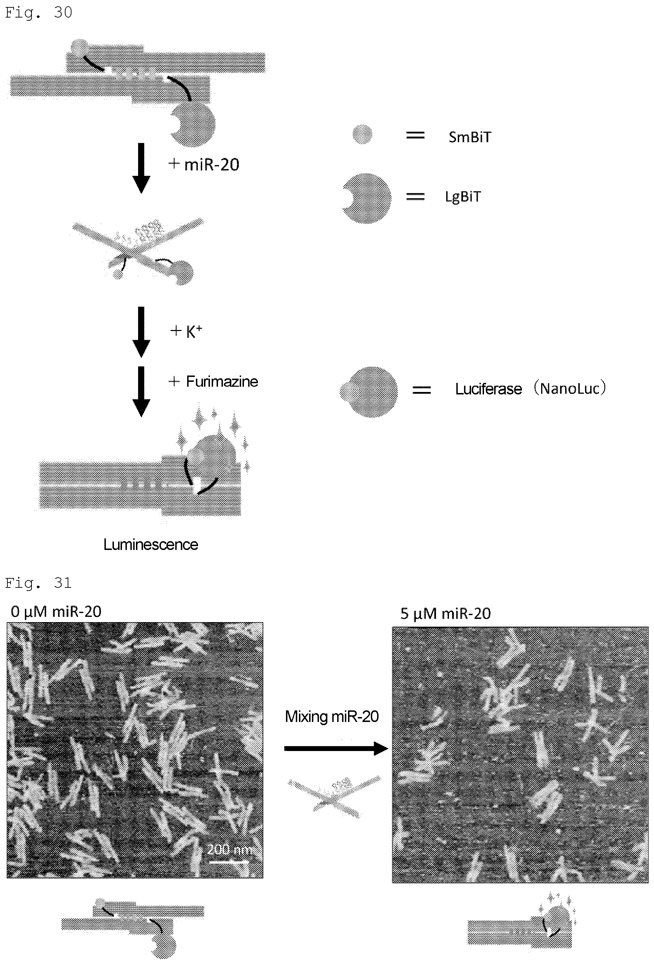

That is, as shown in Fig. 30, the nucleic acid structure of this Example is fixed at the antiparallel type based on complementary binding in the absence of miR-20, whereas in the presence of miR-20, the complementary bond dissociates to induce a structural change to the cross type, and the presence of a monovalent cation induces a structure change to the parallel type based on the formation of a G-quartet structure. As a result, SmBiT and LgBiT are in close proximity and interaction to be reconstituted as a luciferase.

A KCl solution (1 M KCl was dissolved in 1×TAE/Mg²⁺ solution) was added to a solution containing the prepared nucleic acid structure, followed by still standing for 5 minutes. Then, a test substance, miR20 (dissolved in RNase-free Water (catalog number: 9012, TaKaRa) at 200 µM), was added, followed by still standing for 1 hour. AFM observation was then performed, and the presence of miR20 was visualized as a structural difference of the nucleic acid structure. The solution containing the nucleic acid structure contained 3.2 nM of the nucleic acid structure, 5 µM of miR20, and 200 mM of KCl. Further, each structure of the nucleic acid structure was counted from the acquired images, and its proportion was examined.

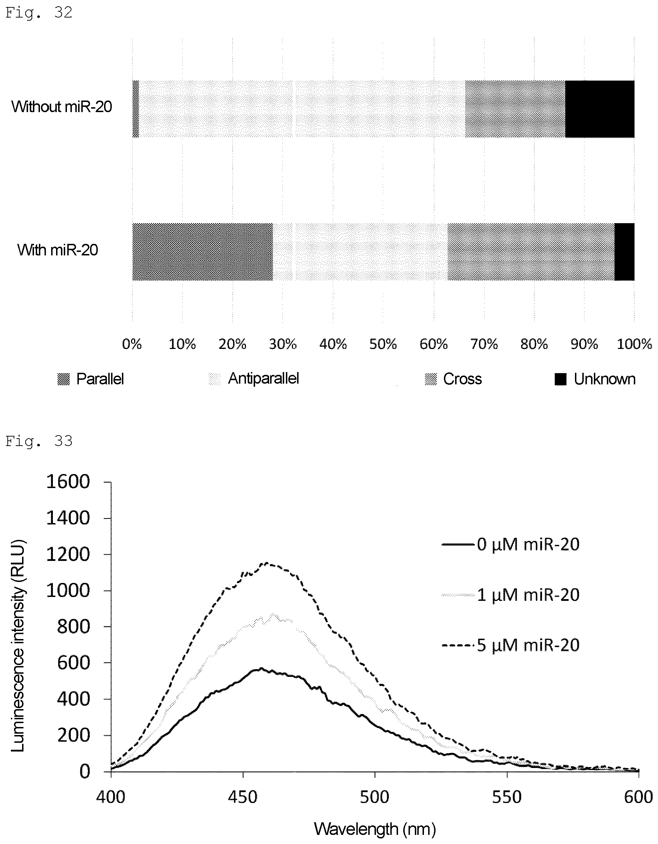

Fig. 31 shows AFM observation images. Further, Fig. 32 shows the proportion of each structure. The antiparallel type was the majority before the addition of miR-20, indicating that the addition of miR-20 induced a structural change from the antiparallel type to the parallel type.

### Example 10: Detection 6 of Enzymatic Activity

A KCl solution (1 M KCl was dissolved in 1×TAE/Mg²⁺ solution) was added to a solution containing the nucleic acid structure (Example 9), followed by still standing for 5 minutes. Then, a test substance, miR20 (dissolved in RNase-free Water (catalog number: 9012, TaKaRa) at 200 µM), was added, followed by still standing for 15 minutes. The solution containing the nucleic acid structure contained 3.2 nM of the nucleic acid structure, 1 µM or 5 µM of miR20, and 200 mM of KCl. Then, furimazine (Nano-Glo Substrate) attached to the Nano-Glo HiBiT Blotting System (Promega) was added, and the luminescence intensity was quantified by a plate reader (SpectraMax iD5, Molecular Devices).

The results are shown in Fig. 33. The luminescence intensity increased when miR-20 was contained. This revealed that miR-20 can be detected using the nucleic acid structure.

SmBiT and LgBiT have a dissociation constant of about 190 µM and extremely low affinity, and they do not express luciferase activity when simply mixed (Andrew, D., (2016) ACS Chem. Biol. 11, 400-408). In the present invention, only when the nucleic acid structure was structurally changed to the parallel type, SmBiT and LgBiT were in close proximity and interaction to succeed in significantly increasing luciferase activity in the presence of a test substance. Further, the luciferase NanoLuc, which is reconstituted by the interaction of SmBiT and LgBiT, is known to have a luminescence intensity about 100 times that of firefly luciferase, and can be photographed with a smartphone camera (Arts, R. et al., (2016) Anal. Chem. 88, 4525-4532). Therefore, visualization is possible by increasing the concentration of the nucleic acid structure.

Sequence Listing

## Claims

1. A nucleic acid structure comprising a partial nucleic acid structure 1 and a partial nucleic acid structure 2,
the partial nucleic acid structure 1 and the partial nucleic acid structure 2 being linked by a Holliday junction,
the partial nucleic acid structure 1 comprising a protruding structure 1a containing a nucleic acid aptamer sequence, and a protruding structure 1b containing a split domain 1 of an enzyme, and
the partial nucleic acid structure 2 comprising a protruding structure 2a containing a sequence complementary to part or all of the nucleic acid aptamer sequence, and a protruding structure 2b containing a split domain 2 paired with the split domain 1.

2. The nucleic acid structure according to claim 1, wherein the partial structure 1 and the partial structure 2 each have an elongated shape.

3. The nucleic acid structure according to claim 1 or 2, which is made of a material containing a single-stranded circular nucleic acid and a staple nucleic acid containing a sequence complementary to the single-stranded circular nucleic acid.

4. The nucleic acid structure according to any one of claims 1 to 3, wherein the protruding structures are arranged so that, among three structures (structural type 1, structural type 2, and structural type 3: provided that the structural type 2 is an intermediate structure that mediates structural changes between the structural type 1 and the structural type 3) based on the conformation of the Holliday junction, when taking the structural type 1, the protruding structure 1a and the protruding structure 2a face each other, and when taking the structural type 3, the protruding structure 1b and the protruding structure 2b face each other.

5. The nucleic acid structure according to claim 4, wherein the protruding structures are arranged so that the protruding structure 1a and the protruding structure 2a face each other and bind together based on complementary base pairing in the absence of a recognition substance for the nucleic acid aptamer sequence.

6. The nucleic acid structure according to claim 4 or 5, wherein the protruding structures are arranged so that the protruding structure 1b and the protruding structure 2b face each other to form the enzyme in the presence of a recognition substance for the nucleic acid aptamer sequence and in the presence of a monovalent cation.

7. The nucleic acid structure according to any one of claims 1 to 6, wherein the enzyme is a nucleic acid enzyme containing two or more G-quartets.

8. The nucleic acid structure according to any one of claims 1 to 7, wherein the recognition substance for the nucleic acid aptamer sequence is a biological material.

9. The nucleic acid structure according to any one of claims 1 to 8, wherein the recognition substance for the nucleic acid aptamer sequence is at least one member selected from the group consisting of nucleic acids, proteins, peptides, low-molecular-weight compounds, physiologically active substances, and metal ions.

10. The nucleic acid structure according to any one of claims 1 to 9, which is a nano-structure.

11. A test substance detection composition comprising the nucleic acid structure according to any one of claims 1 to 10.

12. The test substance detection composition according to claim 11, which is a reagent or a test agent.

13. A method for detecting a test substance, comprising bringing the nucleic acid structure according to any one of claims 1 to 10 into contact with a sample that may contain the test substance.

## Patentansprüche

1. Nukleinsäurestruktur, die eine partielle Nukleinsäurestruktur 1 und eine partielle Nukleinsäurestruktur 2 umfasst,
wobei die partielle Nukleinsäurestruktur 1 und die partielle Nukleinsäurestruktur 2 durch eine Holliday-Struktur verbunden sind,
wobei die partielle Nukleinsäurestruktur 1 eine hervorstehende Struktur 1a, die eine Nukleinsäure-Aptamersequenz enthält, und eine hervorstehende Struktur 1b, die eine Spaltdomäne 1 eines Enzyms enthält, umfasst, und
wobei die partielle Nukleinsäurestruktur 2 eine hervorstehende Struktur 2a, die eine zu einem Teil oder zur Gesamtheit der Nukleinsäure-Aptamersequenz komplementäre Sequenz enthält, und eine hervorstehende Struktur 2b, die eine mit der Spaltdomäne 1 gepaarte Spaltdomäne 2 enthält, umfasst.

2. Nukleinsäurestruktur nach Anspruch 1, wobei die partielle Struktur 1 und die partielle Struktur 2 jeweils eine längliche Form aufweisen.

3. Nukleinsäurestruktur nach Anspruch 1 oder 2, die aus einem Material hergestellt ist, das eine einzelsträngige zirkuläre Nukleinsäure und eine Stapel-Nukleinsäure enthält, die eine zu der einzelsträngigen zirkulären Nukleinsäure komplementäre Sequenz enthält.

4. Nukleinsäurestruktur nach einem der Ansprüche 1 bis 3, wobei die hervorstehenden Strukturen so angeordnet sind, dass unter drei Strukturen (Strukturtyp 1, Strukturtyp 2 und Strukturtyp 3: vorausgesetzt, dass der Strukturtyp 2 eine Zwischenstruktur ist, die strukturelle Veränderungen zwischen dem Strukturtyp 1 und dem Strukturtyp 3 vermittelt), die auf der Konformation der Holliday-Struktur basieren, bei dem Strukturtyp 1 die hervorstehende Struktur 1a und die hervorstehende Struktur 2a einander zugewandt sind und bei dem Strukturtyp 3 die hervorstehende Struktur 1b und die hervorstehende Struktur 2b einander zugewandt sind.

5. Nukleinsäurestruktur nach Anspruch 4, wobei die hervorstehenden Strukturen so angeordnet sind, dass die hervorstehende Struktur 1a und die hervorstehende Struktur 2a einander zugewandt sind und in Abwesenheit einer Erkennungssubstanz für die Nukleinsäure-Aptamersequenz basierend auf komplementärer Basenpaarung miteinander binden.

6. Nukleinsäurestruktur nach Anspruch 4 oder 5, wobei die hervorstehenden Strukturen so angeordnet sind, dass die hervorstehende Struktur 1b und die hervorstehende Struktur 2b einander zugewandt sind, um das Enzym in Gegenwart einer Erkennungssubstanz für die Nukleinsäure-Aptamersequenz und in Gegenwart eines einwertigen Kations zu bilden.

7. Nukleinsäurestruktur nach einem der Ansprüche 1 bis 6, wobei das Enzym ein Nukleinsäureenzym ist, das zwei oder mehr G-Quartette enthält.

8. Nukleinsäurestruktur nach einem der Ansprüche 1 bis 7, wobei die Erkennungssubstanz für die Nukleinsäure-Aptamersequenz ein biologisches Material ist.

9. Nukleinsäurestruktur nach einem der Ansprüche 1 bis 8, wobei die Erkennungssubstanz für die Nukleinsäure-Aptamersequenz mindestens ein Mitglied, ausgewählt aus der Gruppe, bestehend aus Nukleinsäuren, Proteinen, Peptiden, niedermolekularen Verbindungen, physiologisch aktiven Substanzen und Metallionen, ist.

10. Nukleinsäurestruktur nach einem der Ansprüche 1 bis 9, die eine Nanostruktur ist.

11. Testsubstanz-Nachweiszusammensetzung, welche die Nukleinsäurestruktur nach einem der Ansprüche 1 bis 10 umfasst.

12. Testsubstanz-Nachweiszusammensetzung nach Anspruch 11, die ein Reagenz oder ein Testmittel ist.

13. Verfahren zum Nachweis einer Testsubstanz, umfassend das Inkontaktbringen der Nukleinsäurestruktur nach einem der Ansprüche 1 bis 10 mit einer Probe, welche die Testsubstanz enthalten kann.

## Revendications

1. Structure d'acide nucléique comprenant une structure d'acide nucléique partielle 1 et une structure d'acide nucléique partielle 2,
la structure d'acide nucléique partielle 1 et la structure d'acide nucléique partielle 2 étant liées par une jonction de Holliday,
la structure d'acide nucléique partielle 1 comprenant une structure saillante 1a contenant une séquence d'aptamères d'acide nucléique et une structure saillante 1b contenant un domaine divisé 1 d'une enzyme, et
la structure d'acide nucléique partielle 2 comprenant une structure saillante 2a contenant une séquence complémentaire à une partie ou l'ensemble de la séquence d'aptamères d'acide nucléique et une structure saillante 2b contenant un domaine divisé 2 apparié au domaine divisé 1.

2. Structure d'acide nucléique selon la revendication 1, dans laquelle la structure partielle 1 et la structure partielle 2 ont chacune une forme allongée.

3. Structure d'acide nucléique selon la revendication 1 ou 2, qui est constituée d'un matériau contenant un acide nucléique circulaire simple brin et un acide nucléique de base contenant une séquence complémentaire à l'acide nucléique circulaire simple brin.

4. Structure d'acide nucléique selon l'une quelconque des revendications 1 à 3, dans laquelle les structures saillantes sont agencées de sorte que, parmi trois structures (type structurel 1, type structurel 2 et type structurel 3 : à condition que le type structurel 2 soit une structure intermédiaire qui induit des changements structurels entre le type structurel 1 et le type structurel 3) basées sur la conformation de la jonction de Holliday, en prenant le type structurel 1, la structure saillante 1a et la structure saillante 2a se font face, et en prenant le type structurel 3, la structure saillante 1b et la structure saillante 2b se font face.

5. Structure d'acide nucléique selon la revendication 4, dans laquelle les structures saillantes sont agencées de sorte que la structure saillante 1a et la structure saillante 2a se font face et se lient ensemble en fonction d'un appariement de bases complémentaires en l'absence d'une substance de reconnaissance pour la séquence d'aptamères d'acide nucléique.

6. Structure d'acide nucléique selon la revendication 4 ou 5, dans laquelle les structures saillantes sont agencées de sorte que la structure saillante 1b et la structure saillante 2b se font face pour former l'enzyme en présence d'une structure de reconnaissance pour la séquence d'aptamères d'acide nucléique et en présence d'un cation monovalent.

7. Structure d'acide nucléique selon l'une quelconque des revendications 1 à 6, dans laquelle l'enzyme est une enzyme d'acide nucléique contenant deux G-quadruplets ou plus.

8. Structure d'acide nucléique selon l'une quelconque des revendications 1 à 7, dans laquelle la substance de reconnaissance pour la séquence d'aptamères d'acide nucléique est un matériau biologique.

9. Structure d'acide nucléique selon l'une quelconque des revendications 1 à 8, dans laquelle la substance de reconnaissance pour la séquence d'aptamères d'acide nucléique est au moins un élément sélectionné parmi le groupe constitué d'acides nucléiques, de protéines, de peptides, de composés à faible poids moléculaire, de substances physiologiquement actives et d'ions métalliques.

10. Structure d'acide nucléique selon l'une quelconque des revendications 1 à 9, qui est une nanostructure.

11. Composition de détection de substance d'essai comprenant la structure d'acide nucléique selon l'une quelconque des revendications 1 à 10.

12. Composition de détection de substance d'essai selon la revendication 11, qui est un réactif ou un agent d'essai.

13. Procédé de détection d'une substance d'essai, comprenant mettre la structure d'acide nucléique selon l'une quelconque des revendications 1 à 10 en contact avec un échantillon qui peut contenir la substance d'essai.
